(19) Europäisches Patentamt
European Patent Office
Office européen des brevets

(11) EP 4 197 599 A1

(12) **EUROPEAN PATENT APPLICATION**
published in accordance with Art. 153(4) EPC

(43) Date of publication:
**21.06.2023 Bulletin 2023/25**

(21) Application number: **21855994.6**

(22) Date of filing: **12.08.2021**

(51) International Patent Classification (IPC):
***A61Q 19/00*** *(2006.01)* ***A61Q 19/08*** *(2006.01)*
***A61K 8/02*** *(2006.01)* ***A61K 8/19*** *(2006.01)*
***A61K 8/34*** *(2006.01)* ***A61K 8/35*** *(2006.01)*
***A61K 8/81*** *(2006.01)*

(52) Cooperative Patent Classification (CPC):
**A61K 8/02; A61K 8/19; A61K 8/34; A61K 8/35; A61K 8/81; A61Q 19/00; A61Q 19/08**

(86) International application number:
**PCT/JP2021/029753**

(87) International publication number:
**WO 2022/034915 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.08.2020 US 202063065818 P**

(71) Applicant: **Kao Corporation**
**Chuo-ku**
**Tokyo 103-8210 (JP)**

(72) Inventors:
- **HACHIYA, Akira**
  **Cincinnati Ohio 45214 (US)**
- **STRUEWING, Sharon**
  **Cincinnati Ohio 45214 (US)**
- **UCHIYAMA, Masayuki**
  **Tokyo 131-0044 (JP)**
- **NAGASAWA, Hidehiro**
  **Tokyo 131-0044 (JP)**

(74) Representative: **Hoffmann Eitle**
**Patent- und Rechtsanwälte PartmbB**
**Arabellastraße 30**
**81925 München (DE)**

(54) **METHOD FOR PRODUCING FILM**

(57) Provided is an excellent means for reducing wrinkles on skin.

A method for producing a coating film on skin comprising
(Step IB) applying the following composition (B) to the skin and
(Step II) applying a coating film composed of a deposit containing a fiber to the skin;
in this order or in the reverse order:
Composition (B): a composition comprising carbon dioxide gas.

EP 4 197 599 A1

## Description

### Field of the Invention

The present invention relates to a method for producing a coating, and the like.

### Background of the Invention

The intercellular lipid adopts a lamellar structure among the corneocytes, and by this lamellar structure, the moisturizing barrier function is exerted. Various cosmetics having a lamellar structure similar to an intercellular lipid have been proposed so far, and as a technique for forming a lamellar structure, for example, a technique using an amphiphilic molecule such as a ceramide or a fatty acid arginine salt has been known (Patent Document 1, Non Patent Document 1).

For the purpose of obtaining a good feeling of use and improving the blood circulation promoting effect, it has been proposed to incorporate carbon dioxide into a cosmetic (Patent Document 2).

Recently, a technique has been proposed in which a coating film made of a fiber deposit by electrostatic spraying or the like, or a nanofiber sheet having a gradation region in which thickness gradually increases inwardly from a peripheral edge is applied to the skin (Patent Document 3, Patent Document 4).

(Patent Document 1) JP-A-H6-345633
(Patent Document 2) JP-A-2012-167061
(Patent Document 3) JP-A-2018-87186
(Patent Document 4) JP-A-2020-90097

(Non Patent Document 1) J. Oleo. Sci., vol. 54 (6), 325-333 (2005)

### Summary of the Invention

The present invention provides the following <1> to <5>.

<1> A method for producing a coating film on skin, the method comprising

(Step IB) applying the following composition (B) onto the skin and
(Step II) applying a coating film composed of a deposit containing a fiber to the skin,
in this order or in the reverse order:
Composition (B): a composition comprising carbon dioxide gas.

<2> A coating film for application to skin comprising a deposit containing a fiber, and carbon dioxide gas.
<3> A kit for forming a coating film comprising the above composition (B) and a coating film composed of a deposit containing a fiber or a composition for forming a coating film.
<4> A method for reducing wrinkles on skin, the method comprising

(Step I) applying one or two compositions selected from the following compositions (A) and (B) to the skin, and
(Step II) applying a coating film composed of a deposit containing a fiber to the skin
in this order or in the reverse order;
Composition (A): a composition comprising an amphiphilic molecule and having a lamellar structure;
Composition (B): a composition comprising carbon dioxide gas.

<5> Use of a combination of one or two compositions selected from the group consisting of the above compositions (A) and (B), and a coating film composed of a deposit containing a fiber or a composition for forming a coating film, for reducing wrinkles on skin.

### Brief Description of Drawings

Fig. 1 is a schematic diagram showing a configuration of an electrostatic spraying device suitably used in the present invention.

Fig. 2 is a schematic diagram showing a mode of implementing an electrostatic spraying method using an electrostatic spraying device.
Fig. 3 is a diagram showing the change in the area shown by the total of the lines of all wrinkles detected in Test Example 1.
Fig. 4 is a diagram showing the change in the area shown by the total of lines of wrinkles having a thickness of 0.28 mm or more.
Fig. 5 is a diagram showing the change in the area shown by the total of lines of wrinkles ranging from 0.28 to 0.34 mm in thickness.
Fig. 6 is a diagram showing the change in the score of the small wrinkle (fine line) state.
Fig. 7 is a diagram showing an effect of reducing wrinkles when skincare is conducted by the method of Example 3.
Fig. 8 is a diagram showing an effect of reducing wrinkles when skincare is conducted by the method of Comparative Example 3.

Detailed Description of the Invention

Wrinkle is one of skin aging phenomena caused by aging, exposure to UV radiation, or the like. There is a demand for making large wrinkles (coarse lines) or small wrinkles (fine lines) as inconspicuous as possible. Wrinkles from below the eye to the end of the eye are particularly desired to be reduced. It is also desired to provide a means for reducing wrinkles which are hard to remove simply by moisturizing.
The present invention relates to providing an excellent means for effectively reducing wrinkles on skin.
The present inventors found that a coating film obtained on the skin by a method including a step of applying one or two compositions selected from a composition containing an amphiphilic molecule and having a lamellar structure and a composition containing carbon dioxide gas to the skin, and a step of applying a coating film composed of a deposit containing a fiber to the skin in this order or in the reverse order, to complete the present invention.
The method for producing a coating film of the present invention and the kit for forming a coating film facilitate forming a coating film on the skin, which is excellent in the effect of reducing wrinkles on the skin.
Therefore, the coating film of the present invention is excellent in the effect of reducing wrinkles on the skin. The method for reducing wrinkles and the use for reducing wrinkles are excellent in the effect of reducing wrinkles on the skin.

[Method for reducing wrinkles, method for producing a coating film]

The method for reducing wrinkles on skin according to the present invention comprises

(Step I) applying one or two compositions selected from the above compositions (A) and (B) to the skin; and
(Step II) applying a coating film composed of a deposit containing a fiber to the skin
in this order or in the reverse order.

The method for producing a coating film on the skin according to the present invention comprises

(Step IB) applying the above composition (B) to the skin; and
(Step II) applying a coating film composed of a deposit containing a fiber to the skin
in this order or in the reverse order.

In the present specification, a step of applying one or two compositions selected from the compositions (A) and (B) to the skin is referred to as a step I. In the step I, a step of applying the composition (A) to the skin is referred to as a step IA, and a step of applying the composition (B) to the skin is referred to as a step IB.
First, the step IA and the step IB will be described in detail.

-Step IA-

(Composition (A))

The composition (A) is a composition containing an amphiphilic molecule (hereinafter, also referred to as component (a1)) and having a lamellar structure, but is a concept excluding a composition containing carbon dioxide gas, a coating film composed of a deposit containing a fiber, and a composition for forming a coating (e.g., the composition X described hereafter) on which the coating film is formed. In the present invention, "having a lamellar structure" means that an applied coating film at 25°C has a lamellar structure. The lamellar structure can be confirmed by analyzing a

phase state of a cosmetic film by small angle X-ray diffraction measurement and observing a repetitive diffraction peak characteristic of a lamellar structure in the obtained X-ray diffraction profile.

(Component (a1))

An amphiphilic molecule contained in the composition (A), includes for example, ceramides (hereinafter, also referred to as component (a1-1)), polyol fatty acid esters having an intramolecular hydroxy group (hereinafter, also referred to as component (a1-2)), surfactants (hereinafter, also referred to as component (a1-3)), higher alcohols (hereinafter, also referred to as component (a1-4)), sphingosines (e.g., sphingosine, sphingosine salt, pseudosphingosine, pseudosphingosine salt), phospholipids, glycolipids (e.g., glyceroglycolipid, sphingolipid), biosurfactants, sterols (e.g., cholesterol, cholesterol fatty acid ester, cholesterol sulfates, and the like. These may be used alone, or two or more may be used in combination.

Among these, ceramides, polyol fatty acid esters having an intramolecular hydroxy group, a surfactant, and a higher alcohol are preferable from the viewpoint of wrinkle reducing effect, and a combination of one or more kinds selected from the group consisting of ceramides, polyol fatty acid esters having an intramolecular hydroxy group and surfactants, and a higher alcohol is more preferable, and a combination of the components (a1-1) to (a1-4) is particularly preferable.

From the viewpoint of wrinkle reducing effect and the like, the total content of the component (a1) is preferably 0.1 mass% or more, more preferably 0.3 mass% or more, even more preferably 0.5 mass% or more, and particularly preferably 0.7 mass% or more in the composition (A), and from the viewpoint of wrinkle reducing effect and the like, preferably 15 mass% or less, more preferably 12 mass% or less, even more preferably 9 mass% or less, particularly preferably 5 mass% or less in the composition (A). Specifically, the total content of the component (a1) in the composition (A) is preferably 0.1 mass% or more and 15 mass% or less, more preferably 0.3 mass% or more and 12 mass% or less, even more preferably 0.5 mass% or more and 9 mass% or less, and particularly preferably 0.7 mass% or more and 5 mass% or less.

(Components (a1-1))

Examples of the ceramides include those represented by the general formula (4)

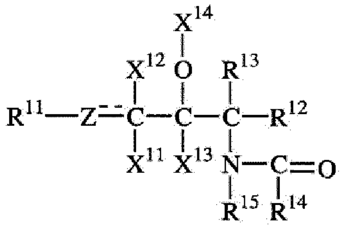

(4)

In Formula (4), $R^{11}$ denotes a straight, branched or cyclic, saturated or unsaturated C4-30 hydrocarbon which may be substituted by hydroxy, carbonyl or amino, or a hydrogen atom;

Z denotes methylene, methine or oxygen atom;
$X^{11}$, $X^{12}$ and $X^{13}$ each independently denote a hydrogen atom, hydroxy, or acetoxy,
$X^{14}$ denotes a hydrogen atom, acetyl or glyceryl, or is taken together with the adjacent oxygen atom to form an oxo (provided that when Z is methine, either $X^{11}$ or $X^{12}$ is a hydrogen atom and the other is absent. When $X^{14}$ forms an oxo, $X^{13}$ is not present;
$R^{12}$ and $R^{13}$ each independently denotes a hydrogen atom, hydroxy, hydroxyalkyl (e.g., hydroxymethyl, hydroxyethyl, or the like) or acetoxymethyl;
$R^{14}$ denotes a straight, branched or cyclic, saturated or unsaturated C5-60 hydrocarbon which may be substituted by hydroxy, carbonyl or amino and which may have ether, ester or amide linkages in the backbone;
$R^{15}$ represents a hydrogen atom or a straight or branched saturated or unsaturated hydrocarbon having 1 to 30 total carbon atoms which may be substituted by hydroxy, hydroxyalkoxy, alkoxy and acetoxy (in addition, when $R^{11}$ is a hydrogen atom and Z is an oxygen atom, $R^{15}$ is preferably hydrocarbon having 10 to 30 total carbon atoms which may be substituted by hydroxy, hydroxyalkoxy, alkoxy and acetoxy; and when $R^{11}$ is hydrocarbon, $R^{15}$ is preferably a hydrogen atom or hydrocarbon having 1 to 8 total carbon atoms which may be substituted by hydroxy, hydroxyalkoxy, alkoxy or acetoxy;
The dashed lines indicate that they may be unsaturated bonds.

In Formula (4), "a linear, branched or cyclic saturated or unsaturated C4-30 hydrocarbon group, in which a hydroxyl group, a carbonyl group or an amino group is optionally substituted" represented by $R^{11}$, is preferably a linear, branched or cyclic saturated or unsaturated C7-22 hydrocarbon group which may be substituted by hydroxy.

Z denotes methylene, methine or an oxygen atom.

$X^{11}$, $X^{12}$ and $X^{13}$ each independently represent a hydrogen atom, hydroxy, or acetoxy. Preferable combinations of $X^{11}$, $X^{12}$ and $X^{13}$ include those in which from 0 to 1 of $X^{11}$, $X^{12}$ and $X^{13}$ is hydroxy and the remainder is a hydrogen atom. When Z is methine, only one of $X^{11}$ and $X^{12}$ is a hydrogen atom and the other is absent.

$X^{14}$ is preferably a hydrogen atom or glyceryl.

Each of $R^{12}$ and $R^{13}$ each independently represent a hydrogen atom, hydroxy, hydroxyalkyl (e.g., hydroxymethyl, hydroxyethyl, or the like) or acetoxymethyl, and $R^{12}$ is preferably a hydrogen atom or hydroxymethyl, and $R^{13}$ is preferably a hydrogen atom.

$R^{14}$ denotes a straight, branched or cyclic saturated or unsaturated C5-60 hydrocarbon which may be substituted by hydroxy, carbonyl or amino and which may have an ether bond, an ester bond or an amide bond in the main chain. $R^{14}$ include straight, branched or cyclic saturated or unsaturated C5-35 hydrocarbon groups which may be substituted by hydroxy or amino, or ester- or amide-bonded straight, branched or cyclic saturated or unsaturated C8-22 fatty acid which may be substituted by hydroxy at the ω-position of the hydrocarbon group. As the fatty acid to be bonded, isostearic acid, 12-hydroxystearic acid or linoleic acid is preferable.

$R^{15}$ denotes a hydrogen atom, or a straight or branched saturated or unsaturated hydrocarbon having 1 to 30 total carbon atoms which may be substituted by hydroxy, hydroxyalkoxy, alkoxy or acetoxy.

When $R^{11}$ is a hydrogen atom and Z is an oxygen atom, $R^{15}$ is preferably a hydrocarbon having 10 to 30 total carbon atoms which may be substituted by hydroxy, hydroxyalkoxy, alkoxy or acetoxy. When $R^{11}$ is a hydrocarbon, $R^{15}$ denotes a hydrogen atom or hydrocarbon having 1 to 8 total carbon atoms which may be substituted by hydroxy, hydroxyalkoxy, alkoxy or acetoxy is preferable, and a hydrogen atom or a hydrocarbon having 1 to 8 total carbon atoms which may be substituted by 1 to 3 of hydroxy, hydroxyalkoxy and alkoxy is more preferable. Here, the hydroxyalkoxy and the alkoxy preferably have 1 to 7 carbon atoms.

As the ceramides, a synthetic product of a natural origin or a similar structure represented by the following general formula (5) and a derivative thereof (hereinafter, also referred to as a natural type ceramide) or a pseudo type ceramide represented by the following general formula (6) (hereinafter, also referred to as a pseudo type ceramide) is preferable. Among natural ceramides and pseudo-type ceramides, pseudo-type ceramides are preferable from the viewpoint of productivity and the like.

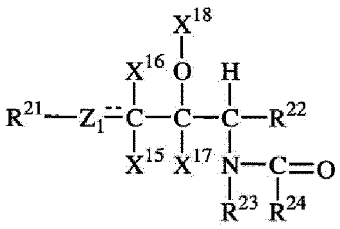

(5)

In Formula (5), $R^{21}$ denotes a straight, branched or cyclic saturated or unsaturated C7-19 hydrocarbon which may be substituted by hydroxy;

$Z_1$ denotes a methylene or a methine;

$X^{15}$, $X^{16}$ and $X^{17}$ each independently represent a hydrogen atom, hydroxy, or acetoxy;

$X^{18}$ denotes a hydrogen atom or forms an oxo together with the adjacent oxygen atom (provided that when $Z_1$ is a methine group, either $X^{15}$ or $X^{16}$ is a hydrogen atom and the other is not present; when $X^{18}$ forms an oxo, $X^{17}$ is not present);

$R^{22}$ denotes a hydroxyalkyl group (e.g., hydroxymethyl, hydroxyethyl, etc.) or acetoxymethyl;

$R^{23}$ denotes a hydrogen atom or C1-4 alkyl;

$R^{24}$ denotes a straight, branched or cyclic saturated or unsaturated C5-30 hydrocarbon which may be substituted by hydroxy (the hydrocarbon group is preferably an alkyl), or an ester linkage of a straight or branched saturated or unsaturated C8-22 fatty acid in which a hydroxy group may be substituted at the ω-terminus of the hydrocarbon (the hydrocarbon is preferably an alkyl);

The dashed lines indicate that they may be unsaturated bonds.

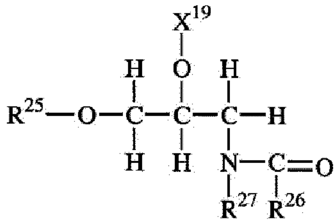

(6)

In Formula (6), $R^{25}$ denotes a straight, branched or cyclic saturated or unsaturated C10-22 hydrocarbon group which may be substituted by a hydroxy group;

$X^{19}$ denotes a hydrogen atom, acetyl or glyceryl;
$R^{26}$ denotes a straight, branched or cyclic, saturated or unsaturated C5-22 hydrocarbon group which may be substituted by hydroxy or amino (the hydrocarbon group is preferably an alkyl), or an esterified straight or branched, saturated or unsaturated C8-22 fatty acid which may be substituted by hydroxy at the omega-terminus of the hydrocarbon group (hydrocarbon group is preferably an alkyl);
$R^{27}$ denotes a hydrogen atom or a hydrocarbon group having 1 to 30 total carbon atoms (the hydrocarbon group is preferably an alkyl group) which may be substituted by hydroxy, hydroxyalkoxy, alkoxy or acetoxy.

Here, a natural ceramide of Formula (5) will be described.
In Formula (5), the number of carbon atoms of the hydrocarbon group of $R^{21}$ is 7 to 19, preferably 11 to 15, more preferably 13 to 15. As the hydrocarbon group of $R^{21}$, a linear alkyl is preferable.
$X^{18}$ denotes a hydrogen atom or is taken together with an adjacent oxygen atom to form an oxo.
$R^{24}$ is preferably a linear C9-27 alkyl group which may be substituted by hydroxy, or a group in which a linoleic acid is esterified to an ω-terminal of the alkyl group. $R^{24}$ is preferably tricosyl, 1-hydroxypentadecyl, 1-hydroxytricosyl, heptadecyl, 1-hydroxyundecyl, and nonacosyl or pentacosyl having a linoleic acid ester-bonded to the ω-position.
Specific examples of the natural type ceramide include, cer amide Types 1 to 7 in which sphingosine, dihydrosphingosine, phyt osphingosine, or sphingadienine is amidated (for example, ceramid es of pig and human described in Fig. 2 of J. Lipid Res., 24: 759 (1983), and Fig. 4 of J. Lipid. Res., 35: 2069 (1994)).
Furthermore, N-alkyl products (for example, an N- methyl pr oduct) of them are included.
As such a ceramide, a natural type (D(-) product) optically -active substance, a non-natural type (L(+) product) optically-ac tive substance, furthermore, a mixture of the natural type and th e non-natural type, may be used. The relative configuration of t he above-mentioned compound may be configuration of a natural typ e, and configuration of the other non-natural type, and further a configuration of a mixture thereof. In particular, CERAMIDE1, C ERAMIDE2, CERAMIDE3, CERAMIDE5, and CERAMIDE6II (all are describe d in INCI, 8th Edition) and compounds represented by the followin g formula are preferable.

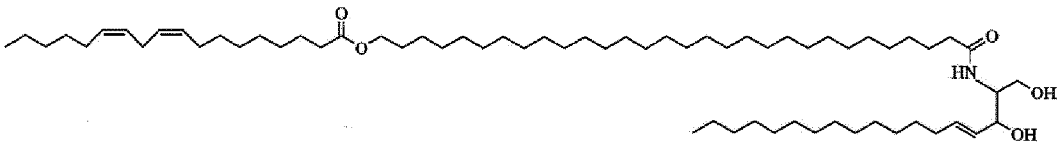

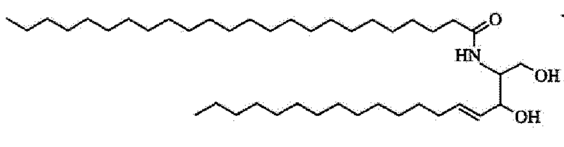

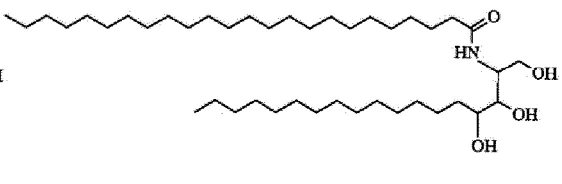

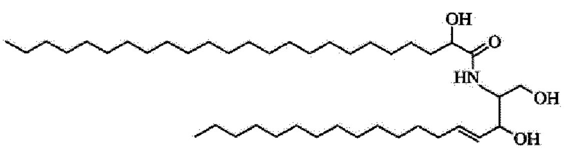

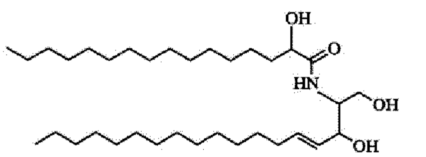

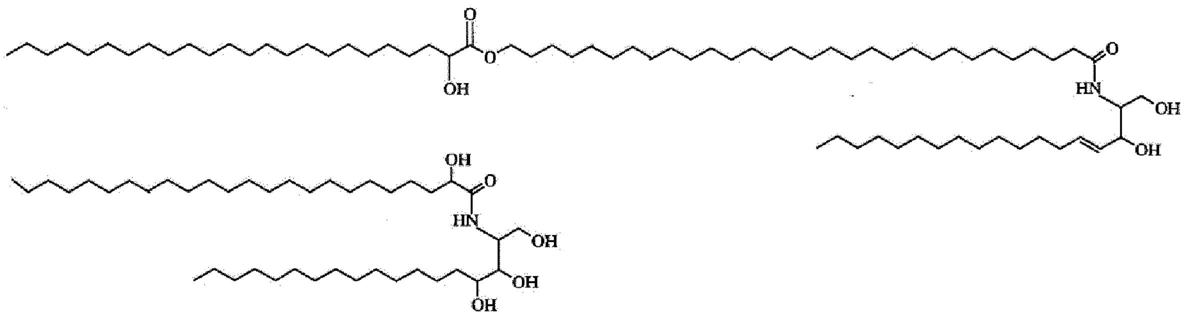

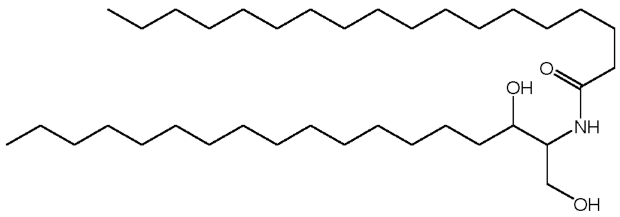

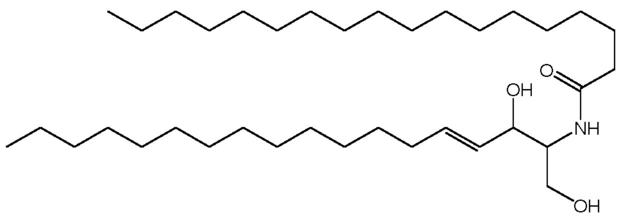

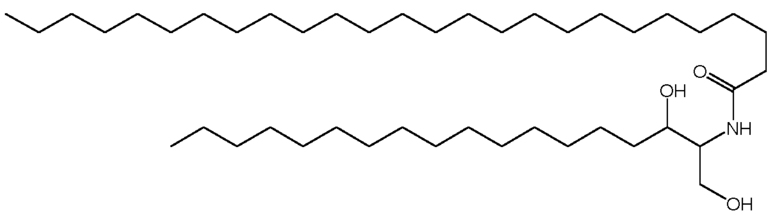

These may be either a naturally extracted product or a synt hesized product, and commercially available products can be used.

When such commercially available natural type ceramides are used, preferred are one or two or more selected from Ceramide I, Ceramide III, Ceramide IIIA, Ceramide IIIB, Ceramide IIIC, Cerami de VI (all manufactured by Cosmoferm Co., Ltd.), Ceramide TIC-001 (manufactured by TAKASAGO INTERNATIONAL CORPORATION), CERAMIDE I I (manufactured by Quest International Co., Ltd.), DS-Ceramide V I, DS-CLA-Phytoceramide, C6-Phytoceramide, DS-ceramide Y3S (manu factured by DOOSAN Co., Ltd.), CERAMIDE2 (manufactured by Sederma Co., Ltd.).

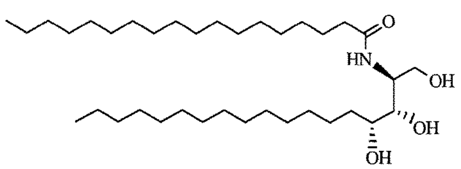

Ceramide III (manufactured by Cosmoferm Co., Ltd.)

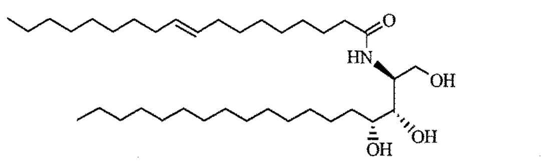

Ceramide IIIB (manufactured by Cosmoferm Co., Ltd.)

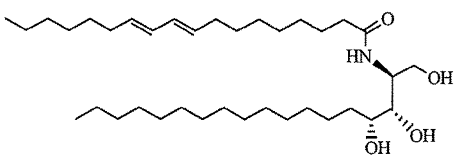

Ceramide IIIA (manufactured by Cosmoferm Co., Ltd.)

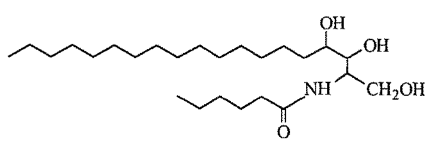

Phytoceramide (manufactured by DOOSAN Co., Ltd.)

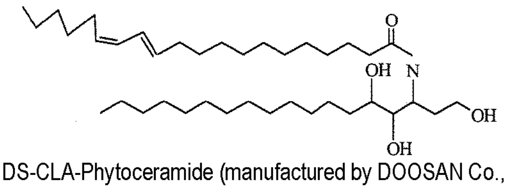

DS-CLA-Phytoceramide (manufactured by DOOSAN Co., Ltd.)

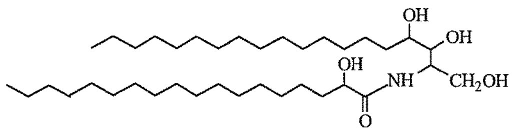

DS-Ceramide VI (manufactured by DOOSAN Co., Ltd.)

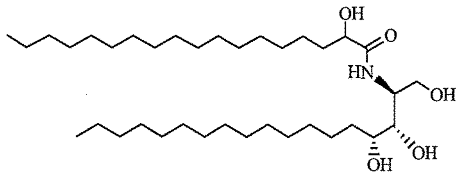

Ceramide IV (manufactured by Cosmoferm Co., Ltd.)

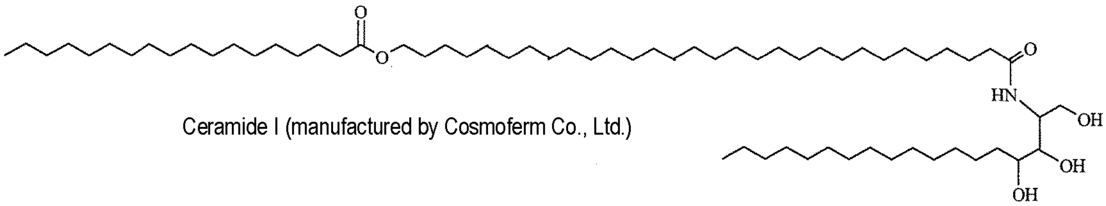

Ceramide I (manufactured by Cosmoferm Co., Ltd.)

Next, a pseudo-type ceramide of formula (6) will be described.

In Formula (6), the preferable $R^{26}$ are heptyl, 1-hydroxyheptyl, nonyl, tridecyl, pentadecyl, undecyl with linoleic acid esterified in ω position, pentadecyl with linoleic acid esterified in ω position, pentadecyl with 12-hydroxystearic acid esterified in ω position, and undecyl with methyl branched isostearic acid amide linked in ω position.

When $R^{25}$ is a hydrogen atom, as $R^{27}$, a hydrocarbon having 1 to 30 total carbon atoms (the hydrocarbon is preferably an alkyl) which may be substituted by hydroxyl, hydroxyalkoxy, alkoxy or acetoxy is preferable, and a hydrocarbon having 10 to 30 total carbon atoms (a hydrocarbon group is preferably alkyl) which may be substituted by hydroxyl, hydroxyalkoxy, alkoxy or acetoxy is more preferable, and alkyl having 12 to 20 total carbon atoms is particularly preferable.

When $R^{25}$ is a straight, branched or cyclic saturated or unsaturated C10-22 hydrocarbon group which may be substituted by hydroxy, $R^{27}$ is preferably a hydrogen atom or an alkyl having 1 to 8 total carbon atoms which may be substituted by hydroxyl, hydroxyalkoxy, alkoxy or acetoxy.

The hydroxyalkoxy or alkoxy in $R^{27}$ preferably has 1 to 7 carbon atoms.

Among the pseudo-type ceramides, in Formula (6), a compound in which $R^{25}$ is hexadecyl, $X^{19}$ is a hydrogen atom, $R^{26}$ is pentadecyl, and $R^{27}$ is hydroxyethyl; a compound in which $R^{25}$ is hexadecyl, $X^{19}$ is a hydrogen atom, $R^{26}$ is nonyl, and $R^{27}$ is hydroxyethyl; a compound in which $R^{25}$ is hexadecyl, $X^{19}$ is glyceryl, $R^{26}$ is tridecyl, and $R^{27}$ is 3-methoxypropyl; a compound in which $R^{25}$ is a hydrogen atom, $X^{19}$ is a hydrogen atom, $R^{26}$ is pentadecyl, and $R^{27}$ is dodecyl; and a compound in which $R^{25}$ is a hydrogen atom, $X^{19}$ is a hydrogen atom, $R^{26}$ is 1-hydroxyheptyl, and $R^{27}$ is dodecyl are preferable.

More preferable are, in Formula (6), a compound in which $R^{25}$ is hexadecyl, $X^{19}$ is a hydrogen atom, $R^{26}$ is pentadecyl, and $R^{27}$ is hydroxyethyl; a compound in which $R^{25}$ is hexadecyl, $X^{19}$ is a hydrogen atom, $R^{26}$ is nonyl, and $R^{27}$ is hydroxyethyl; and a compound in which $R^{25}$ is hexadecyl, $X^{19}$ is glyceryl, $R^{26}$ is tridecyl, and $R^{27}$ is 3-methoxypropyl.

Particular preference is given to those in formula (6), in which $R^{25}$ is hexadecyl, $X^{19}$ is a hydrogen atom, $R^{26}$ is pentadecyl, and $R^{27}$ is hydroxyethyl (N-(hexadecyloxyhydroxypropyl)-N-hydroxyethylhexadecanamide) .

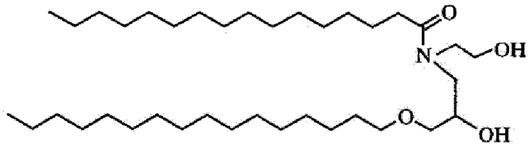

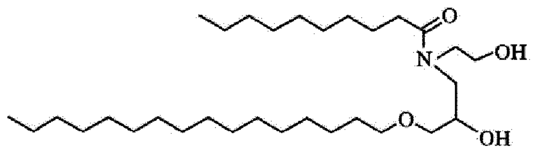

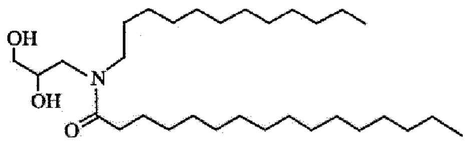

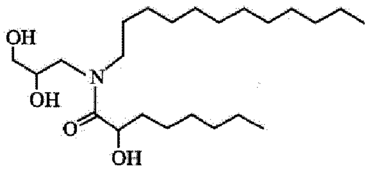

The ceramides of the component (a1-1) may be used alone, or two or more kinds thereof may be used in combination.

From the viewpoint of wrinkle reducing effect and the like, the content of the component (a1-1) in the composition (A) is preferably 0.001 mass% or more, more preferably 0.01 mass% or more, even more preferably 0.1 mass% or more, particularly preferably 0.2 mass% or more, and from the viewpoint of wrinkle reducing effect and the like, preferably 10 mass% or less, more preferably 7 mass% or less, even more preferably 5 mass% or less, and particularly preferably 3 mass% or less. Specifically, the content of the component (a1-1) in the composition (A) is preferably 0.001 mass% or more and 10 mass% or less, more preferably 0.01 mass% or more and 7 mass% or less, even more preferably 0.1 mass% or more and 5 mass% or less, and particularly preferably 0.2 mass% or more and 3 mass% or less.

When the component (a1-1) and the component (a1-4) are used in combination, the content mass ratio of the component (a1-1) to the component (a1-4), [(a1-1)/ (a1-4)], is preferably 0.2 or more, more preferably 0.23 or more, even more preferably 0.26 or more, particularly preferably 0.3 or more, from the viewpoint of wrinkle reducing effect and the like, and is preferably 5 or less, more preferably 4 or less, even more preferably 3.5 or less, and particularly preferably 3 or less from the viewpoint of wrinkle reducing effect and the like. Specifically, [(a1-1)/ (a1-4)] is preferably 0.2 or more and 5 or less, more preferably 0.23 or more and 4 or less, even more preferably 0.26 or more and 3.5 or less, and particularly preferably 0.3 or more and 3 or less.

(Components (a1-2))

Examples of the polyol fatty acid ester having an intramolecular hydroxy group include glycerol monofatty acid esters, glycerol difatty acid esters, sorbitan monofatty acid esters, sorbitan difatty acid esters, and diglycerol difatty acid esters. Among these, glycerol monofatty acid esters, sorbitan difatty acid esters, and diglycerin difatty acid esters are preferable, and glycerol monofatty acid esters and diglycerol difatty acid esters are more preferable.

As the fatty acid residue of the polyol fatty acid ester having an intramolecular hydroxyl group, a residue of a C10-24 fatty acid is preferable, a residue of a C16-24 fatty acid is more preferable, and a residue of a C18-24 fatty acid is particularly preferable. The fatty acid residue may be a saturated fatty acid residue or an unsaturated fatty acid residue, or may be a straight chain fatty acid residue or a branched fatty acid residue. As the polyhydric alcohol fatty acid esters having an intramolecular hydroxy, those having a residue of a saturated fatty acid are preferable.

Examples of the glycerol monofatty acid esters include glycerol monolaurate, glycerol monomyristate, glycerol monopalmitate, glycerol monostearate, glycerol monobehenate, glycerol monooleate, glycerol monoisostearate, and glycerol monolinolate. Among these, glycerol monostearate, and glycerol monobehenate are preferable.

Examples of the diglycerol difatty acid ester include polyglyceryl diisostearate-2, polyglyceryl dilaurate-2, polyglyceryl distearate-2, and polyglyceryl dioleate-2.

The component (a1-2) may be used alone, or two or more kinds thereof may be used in combination.

From the viewpoint of wrinkle reducing effect and the like, the content of the component (a1-2) in the composition (A) is preferably 0.001 mass% or more, more preferably 0.01 mass% or more, even more preferably 0.05 mass% or more, particularly preferably 0.1 mass% or more, and from the viewpoint of wrinkle reducing effect and the like, preferably 10 mass% or less, more preferably 5 mass% or less, even more preferably 3 mass% or less, and particularly preferably 2 mass% or less in the composition (A). Specifically, in the composition (A), it is preferably 0.001 mass% or more and

10 mass% or less, more preferably 0.01 mass% or more and 5 mass% or less, even more preferably 0.05 mass% or more and 3 mass% or less, and particularly preferably 0.1 mass% or more and 2 mass% or less.

When the component (a1-2) and the component (a1-4) are used in combination, the mass ratio of the component (a1-2) to the component (a1-4), [(a1-2)/ (a1-4)], is preferably 0.1 or more, more preferably 0.13 or more, even more preferably 0.16 or more, particularly preferably 0.2 or more, from the viewpoint of wrinkle reducing effect and the like, and is preferably 5 or less, more preferably 4 or less, even more preferably 3.5 or less, and particularly preferably 3 or less from the viewpoint of wrinkle reducing effect and the like. Specifically, it is preferably 0.1 or more and 5 or less, more preferably 0.13 or more and 4 or less, even more preferably 0.16 or more and 3.5 or less, and particularly preferably 0.2 or more and 3 or less.

(Component (a1-3))

Examples of the surfactant as the component (a1-3) include an ionic surfactant, and a nonionic surfactant other than the component (a1-2). Among these, an ionic surfactant is preferable, and an anionic surfactant, a cationic surfactant (alkyltrimethylammonium chloride such as stearyltrimethylammonium chloride, lauryltrimethylammonium chloride; dialkyldimethylammonium chloride, trialkylmethylammonium chloride, alkylamine salts, and the like), and an amphoteric surfactant (alkyldimethylamine oxide, alkylcarboxy betaine, alkylsulfobetaine, amido amino acid salts, alkylamidopropylbetaine, and the like) are more preferable, and an anionic surfactant is particularly preferable.

Examples of the anionic surfactants include, fatty acid salts such as sodium laurate, potassium palmitate and arginine stearate (preferably C12-24 fatty acid salts); sodium lauryl sulfate and potassium lauryl sulfate, alkyl sulfate salts such as sodium cetyl sulfate (preferably C12-24 alkyl sulfate salts); polyoxyethylene alkyl ether sulfate salts such as polyoxyethylene lauryl ether sulfate triethanolamine (preferably C12-24 alkyl ether sulfate salts); N-acyl sarcosine salts such as sodium lauroyl sarcosine (preferably N-(C12-24 acyl)sarcosine salts); alkyl phosphate salts such as sodium monostearyl phosphate (preferably C12-24 alkyl phosphate sodium salt); and polyoxyethylene oleyl ether phosphate salts, Polyoxyethylene alkyl ether phosphates, such as sodium polyoxyethylene stearyl ether phosphate (preferably C12-24 alkyl ether phosphates); dialkyl sulfosuccinates, such as sodium di(2-ethylhexyl)sulfosuccinate (preferably di(C6-12 alkyl)sulfosuccinates); N-alkyloyl methyl taurate, such as sodium N- stearoyl-N-methyl taurate and sodium N-myristoyl-N-methyl taurate (preferably N-alkyloyl methyl taurate with C12-24); N-acylglutamates such as sodium N-lauroyl-L-glutamate, sodium N-myristoyl-L-glutamate, sodium N-stearoyl-L-glutamate, disodium N-stearoyl-L-glutamate, potassium N-stearoyl-L-glutamate, and N-stearoyl-L-glutamic acid arginine (preferably N-(C12-24 acyl)glutamates) .

Among these anionic surfactants, an N-(C12-24 acyl) glutamate, a C12-24 fatty acid salt, and a polyoxyethylene with C12-24 alkyl ether phosphate are preferable, an N-(C12-24 acyl)glutamate is more preferable, an N-acylglutamate having a C12-20 acyl group is more preferable, and a N-stearoyl-L-glutamate is particularly preferable.

As the nonionic surfactant other than the component (a1-2), a nonionic surfactant having an HLB of 10 or higher and a polyoxyethylene group is preferable, and examples thereof include polyoxyethylene cured castor oil, polyoxyethylene sorbitan fatty acid ester, alkyl polyoxyethylene glyceryl, and polyoxyethylene alkyl ether.

The component (a1-3) may be used alone, or two or more kinds thereof may be used in combination.

From the viewpoint of wrinkle reducing effect and the like, the content of the component (a1-3) in the composition (A) is preferably 0.01 mass% or more, more preferably 0.05 mass% or more, even more preferably 0.075 mass% or more, particularly preferably 0.1 mass% or more, and from the viewpoint of wrinkle reducing effect and the like, preferably 3 mass% or less, more preferably 2.5 mass% or less, even more preferably 2 mass% or less, and particularly preferably 1.5 mass% or less in the composition (A). Specifically, the content of the component (a1-3) in the composition (A) is preferably 0.01 % or more and 3 mass% or less, more preferably 0.05 mass% or more and 2.5 mass% or less, even more preferably 0.075 mass% or more and 2 mass% or less, and particularly preferably 0.1 mass% or more and 1.5 mass% or less.

In the present specification, among the components (a1-3), the content of the anionic surfactant in the composition (A) is shown as a mass in terms of acid. Hereinafter, the same applies to mass ratio of the content of the component (a1-3) and the other components and the like.

When the component (a1-3) and the component (a1-4) are used in combination, the content mass ratio of the component (a1-3) to the component (a1-4), [(a1-3)/ (a1-4)], is preferably 0.1 or more, more preferably 0.13 or more, even more preferably 0.16 or more, particularly preferably 0.2 or more, from the viewpoint of wrinkle reducing effect and the like, and is preferably 5 or less, more preferably 4 or less, even more preferably 3.5 or less, and particularly preferably 3 or less from the viewpoint of wrinkle reducing effect and the like. Specifically, it is preferably 0.1 or more and 5 or less, more preferably 0.13 or more and 4 or less, even more preferably 0.16 or more and 3.5 or less, and particularly preferably 0.2 or more and 3 or less.

(Components (a1-4))

As the higher alcohol as the component (a1-4), a monovalent C10-24 alcohol is preferable, a monovalent C12-22 alcohol is more preferable, and a monovalent C14-22 alcohol is particularly preferable. The higher alcohol may be linear or branched, and may be a saturated alcohol or an unsaturated alcohol, but a linear saturated or unsaturated alcohol is preferable.

Examples of the higher alcohol include lauryl alcohol, myristyl alcohol, cetanol, stearyl alcohol, behenyl alcohol, and oleyl alcohol. Among these, cetanol, stearyl alcohol, and behenyl alcohol are preferable, and cetanol is more preferable.

The higher alcohol as the component (a1-4) may be used alone, or two or more kinds thereof may be used in combination.

From the viewpoint of wrinkle reducing effect and the like, the content of the component (a1-4) in the composition (A) is preferably 0.001 mass% or more, more preferably 0.01 mass% or more, even more preferably 0.1 mass% or more, and particularly preferably 0.5 mass% or more, and from the viewpoint of wrinkle reducing effect and the like, preferably 10 mass% or less, more preferably 5 mass% or less, even more preferably 3 mass% or less, and particularly preferably 2.5 mass% or less in the composition (A). Specifically, the content of the component (a1-4) in the composition (A) is preferably 0.001 mass% or more and 10 mass% or less, more preferably 0.01 mass% or more and 5 mass% or less, even more preferably 0.1 mass% or more and 3 mass% or less, and particularly preferably 0.5 mass% or more and 2.5 mass% or less.

From the viewpoint of wrinkle reducing effect, moisturizing effect, and the like, the composition (A) preferably contains, in addition to the amphiphilic molecule described above, one or more kinds selected from the group consisting of (a2) an oily component other than the component (a1), (a3) a polyol and (a4) water, more preferably contains the components (a1), (a2) and (a4), and particularly preferably contains the components (a1) to (a4).

(Component (a2))

Examples of the (a2) oily component include a hydrocarbon oil, an ether oil, an ester oil, a higher fatty acid, a silicone oil, and a fluorine oil. Specifically, hydrocarbon oils such as α-olefin oligomer, liquid paraffin, squalane, and petrolatum; ether oils such as cetyldimethyl butyl ether, ethylene glycol dioctyl ether, and glycerol monooleyl ether; ester oils such as octyldodecyl myristate, isopropyl palmitate, butyl stearate, di-2-ethylhexyl adipate, neopentyl glycol dicaprate, trioctanoin, and the like; higher fatty acids such as stearic acid, behenic acid, isomyristic acid; silicone oils such as methyl polysiloxane, circular dimethyl polysiloxane, methylphenyl polysiloxane, amino-modified silicone, carboxy-modified silicone, alcohol-modified silicone, alkyl-modified silicone, polyether-modified silicone, fluorine-modified silicone; fluorine-based oils such as perfluoroalkylethyl phosphoric acid, perfluoroalkyl polyoxyethylene phosphoric acid, perfluoropolyether, and polytetrafluoroethylene.

The (a2) oily component is preferably a liquid or semisolid component at 25°C.

The component (a2) may be used alone, or two or more kinds thereof may be used in combination.

Examples of the component (a2) include a vegetable oil such as olive oil or jojoba oil or an animal oil such as deep sea shark oil or lanolin containing the above ester oil or hydrocarbon oil.

The content of the component (a2) in the composition (A) is preferably 0.001 mass% or more, more preferably 0.01 mass% or more, even more preferably 0.15 mass% or more, and particularly preferably 0.2 mass% or more from the viewpoint of wrinkle reducing effect and the like, and is preferably 20 mass% or less, more preferably 17 mass% or less, even more preferably 15 mass% or less, and particularly preferably 13 mass% or less in the composition (A) from the viewpoint of wrinkle reducing effect and the like. Specifically, in the composition (A), 0.001 % or more and 20 mass% or less are preferable, 0.01 mass% or more and 17 mass% or less are more preferable, 0.15 mass% or more and 15 mass% or less are even more preferable, and 0.2 mass% or more and 13 mass% or less are particularly preferable.

When a silicone oil is used as the component (a2), from the viewpoint of adhesion of the coating film to the skin, the content of the silicone oil in the composition (A) is preferably 10 mass% or less, more preferably 7 mass% or less.

When a volatile oily component having a viscosity of less than 10 mPa·s at 30°C is used as the component (a2), the content thereof in the composition (A) is preferably 10 mass% or less, more preferably 5 mass% or less, even more preferably 1 mass% or less, even more preferably 0.5 mass% or less, and may not be contained from the viewpoint of adhesion of the coating film to the skin.

The mass ratio of the component (a2) to the component (a1), [(a2)/(a1)], is preferably 0.1 or more, more preferably 0.13 or more, even more preferably 0.16 or more, particularly preferably 0.2 or more, from the viewpoint of wrinkle reducing effect and the like, and is preferably 5 or less, more preferably 4 or less, even more preferably 3.5 or less, and particularly preferably 3 or less from the viewpoint of wrinkle reducing effect and the like. Specifically, the [(a2)/(a1)] is preferably 0.1 or more and 5 or less, more preferably 0.13 or more and 4 or less, even more preferably 0.16 or more and 3.5 or less, and particularly preferably 0.2 or more and 3 or less.

(Component (a3))

Examples of the (a3) polyol as the component (a3) include glycols and glycerols. Examples of the glycols include alkylene glycols such as ethylene glycol, propylene glycol, 1,3-propanediol, and 1,3-butylene glycol; dialkylene glycols such as diethylene glycol and dipropylene glycol; polyalkylene glycols such as polyethylene glycol (e.g., polyethylene glycol 1000, polyethylene glycol 1540, polyethylene glycol 2000, and the like), and polypropylene glycol. Examples of the glycerols include glycerin, diglycerin, and polyglycerin. The number average molecular weight of the polyalkylene glycol is preferably 300 or more and 15 000 or less, more preferably 400 or more and 10 000 or less, even more preferably 500 or more and 5 000 or less, and particularly preferably 600 or more and 2 500 or less.
Among these polyols, alkylene glycol, polyethylene glycol, and glycerol are preferable, and 1,3-butylene glycol, polyethylene glycol, and glycerin are particularly preferable.
The polyols may be used alone, or two or more kinds thereof may be used in combination.
The content of the component (a3) in the composition (A) is preferably 0.001 mass% or more, more preferably 0.01 mass% or more, even more preferably 0.2 mass% or more, and particularly preferably 2 mass% or more, from the viewpoint of wrinkle reducing effect and the like, and is preferably 30 mass% or less, more preferably 28 mass% or less, even more preferably 26 mass% or less, particularly preferably 25 mass% or less in the composition (A) from the viewpoint of wrinkle reducing effect and the like. Specifically, in the composition (A), it is preferably 0.001 mass% or more and 30 mass% or less, more preferably 0.01 mass% or more and 28 mass% or less, even more preferably 0.2 mass% or more and 26 mass% or less, and particularly preferably 2 mass% or more and 25 mass% or less.
The mass ratio of the component (a3) to the component (a1), [(a3)/(a1)], is preferably 0.2 or more, more preferably 0.23 or more, even more preferably 0.26 or more, particularly preferably 0.3 or more, from the viewpoint of wrinkle reducing effect and the like, and is preferably 10 or less, more preferably 8 or less, even more preferably 6 or less, and particularly preferably 5.5 or less from the viewpoint of wrinkle reducing effect and the like. Specifically, it is preferably 0.2 or more and 10 or less, more preferably 0.23 or more and 8 or less, even more preferably 0.26 or more and 6 or less, and particularly preferably 0.3 or more and 5.5 or less.

(Component (a4))

From the viewpoint of wrinkle reducing effect and the like, the content of water in the composition (A) is preferably 40 mass% or more, more preferably 45 mass% or more, even more preferably 50 mass% or more, and particularly preferably 55 mass% or more, and from the viewpoint of wrinkle reducing effect and the like, it is preferably 90 mass% or less, more preferably 85 mass% or less, even more preferably 80 mass% or less, and particularly preferably 75 mass% or less in the composition (A). Specifically, in the composition (A), it is preferably 40 mass% or more and 90 mass% or less, more preferably 45 mass% or more and 85 mass% or less, even more preferably 50 mass% or more and 80 mass% or less, and particularly preferably 55 mass% or more and 75 mass% or less.
The composition (A) may contain an active ingredient, an additive, and the like other than those commonly used in cosmetics. Examples of such an active ingredient or an additive include, bases such as potassium hydroxide, sodium hydroxide, triethanolamine, sodium carbonate, and arginine; water-soluble vitamins such as ascorbic acid, nicotinamide, and nicotinic acid; animal and plant extracts such as Amur cork tree bark extract, kanzo (*glycyrrhiza*) extract, aloe extract, field horsetail extract, tea plant extract, cucumber extract, clove extract, ginseng extract, hamamelis extract, placenta extract, seaweed extract, horse chestnut extract, chamomilla recutita extract, yuzu (*Citrus junos*) extract, clove extract, hiba arborvitae (*Thujopsis dolabrata*) extract, artea extract, royal jelly extract, eucalyptus extract, brown algae extract, and hiba arborvitae (*Thujopsis dolabrata*) extract; preservatives; pH-adjusting agents; viscosity-adjusting agents such as a carboxyvinyl polymer, sodium alginate, carrageenan, carboxymethyl cellulose, hydroxyethyl cellulose, guar gum, xanthan gum, carboxymethyl chitosan, sodium hyaluronate, oxazoline-modified silicone, N,N-dimethylaminoethyl methacrylate diethyl sulfate - N,N-dimethylacrylamide - dimethacrylate polyethylene glycol copolymer; powders ; perfumes or the like may be included. One of these may be used alone, or two or more may be used in combination.
The composition (A) is preferably an emulsified composition. The type of emulsification thereof is not particularly limited; it can be either oil-in-water or water-in-oil, but an oil-in-water type emulsified composition is preferable. The form of the composition (A) is not particularly limited as long as it is a conventional form as a skincare cosmetic product, and examples thereof include a liquid, an emulsion, a gel, and a cream.
The composition (A) can be produced by an ordinary method.

-Step IB-

(Composition (B))

The composition (B) contains carbon dioxide gas (hereinafter, also referred to as a component (b1)), but the

composition (B) refers to a concept excluding a coating film composed of a deposit containing a fiber and a composition (e.g., a composition X described hereafter) for forming a coating film.

The composition (B) may preferably contain, from the viewpoint of wrinkle reducing effect, blood circulation promoting effect and the like, one or more kinds selected from the group consisting of (b2) an oil agent, (b3) a polyol, (b4) a surfactant, (b5) a water-soluble thickener and (b6) water, more preferably contain the components (b1), (b2), (b4), (b5) and (b6), and particularly preferably the components (b1) to (b6).

(Component (b2))

Oil agents are roughly classified into those in a liquid state at 25°C and those in a state other than liquid at 25°C. Examples of the oil agents in a state other than liquid at 25°C include a wax and a solid fat in a solid state at 25°C.

The oil agent in a liquid state at 25°C is preferably one or more kinds selected from the group consisting of hydrocarbon oils, ester oils, ether oils and silicone oils. Examples of the hydrocarbon oils include liquid paraffin, squalane, and squalene. Examples of the ester oils include mono-ester oils such as isopropyl palmitate, isopropyl stearate, butyl stearate, isopropyl myristate, cetyl 2-ethylhexanoate, 2-ethylhexyl palmitate, 2-octyldodecyl myristate, 2-octyldodecyl oleate, glyceryl triisostearate, isotridecyl isononanoate, (C12-15)alkyl benzoate; di-ester oils or tri-ester oils such as diisopropyl adipate, diisobutyl adipate, neopentyl glycol dicaprate, tri(caprylic/capric acid) glyceryl, glycerin tricaprylate, neopentylglycol diethylhexanoate, di(caprylic/capric acid) propanediol, diisostearyl malate, and di(cholesteryl/octyldodecyl) N-lauroyl-L-glutamate. Examples of the eter oils include dicaprylyl ether. Examples of the silicone oil include methylpolysiloxane, cyclic dimethylpolysiloxane, methylphenylpolysiloxane, amino-modified silicone, carboxy-modified silicone, alcohol-modified silicone, alkyl-modified silicone, polyether-modified silicone, and fluorine-modified silicone.

Examples of the oil agent include vegetable oils such as spruce oil, jojoba oil, and olive oil containing the above-mentioned oils, animal oils such as lanolin, and essential oils such as eucalyptus oil and turnip oil as well.

Among these, ester oils and silicone oils are preferable from the viewpoint of wrinkle reducing effect, impression from use, retention of carbon dioxide in the composition, suppression of volatilization of carbon dioxide gas, and the like, and monoester oil, diester oil, and silicone oil are more preferable, and monoester oil and silicone oil are more preferable, and isotridecyl isononanoate, (C12-15)alkyl benzoate, and methylpolysiloxane are particularly preferable.

The viscosity of the above methylpolysiloxane at 25°C, preferably 6 mPa·s or more and 5 000 mPa·s or less, more preferably 10 mPa·s or more and 1 000 mPa·s or less, and particularly preferably 10 mPa.s or more and 500 mPa.s or less. When two or more kinds of methylpolysiloxanes are used, the viscosity of the mixture may be in the above range.

The viscosity of the above methylpolysiloxane is a value measured using a BM viscometer (manufactured by Toki Sangyo Co., Ltd.) at 25°C.

The component (b2) may be used alone, or two or more kinds thereof may be used in combination.

The content of the component (b2) in the composition (B) is preferably 0.1 mass% or more, more preferably 0.5 mass% or more, and even more preferably 1 mass% or more from the viewpoint of wrinkle reducing effect and the like, and is preferably 20 mass% or less, more preferably 15 mass% or less, and even more preferably 7 mass% or less in the composition (B) from the viewpoint of wrinkle reducing effect and the like. Specifically, in the composition (B), 0.1 mass% or more and 20 mass% or less are preferable, 0.5 mass% or more and 15 mass% or less are more preferable, and 1 mass% or more and 7 mass% or less are even more preferable.

When the composition (B) is an aerosol composition, the content of the component (b2) refers to a proportion of the component (b2), assuming the stock solution to be 100 mass%. Hereinafter, the same applies to other components.

(Component (b3))

Examples of the (b3) polyol include glycols and glycerols. Examples of the glycols include alkylene glycols such as ethylene glycol, propylene glycol, 1,3-propanediol, and 1,3-butylene glycol; dialkylene glycols such as diethylene glycol and dipropylene glycol; polyalkylene glycols such as polyethylene glycol (e.g., polyethylene glycol 1000, polyethylene glycol 1540, polyethylene glycol 2000, and the like), and polypropylene glycol. Examples of the glycerols include glycerin, diglycerin, and polyglycerin. The number average molecular weight of the polyalkylene glycol is preferably 300 or more and 15 000 or less, more preferably 400 or more and 10 000 or less, even more preferably 500 or more and 5 000 or less, and particularly preferably 600 or more and 2 500 or less.

Among these polyols, alkylene glycols, dialkylene glycols, and polyethylene glycols are preferable, and 1,3-propanediol, dipropylene glycol, and polyethylene glycol are particularly preferable.

The polyol may be used alone, or two or more kinds thereof may be used in combination.

The content of the component (b3) in the composition (B) is preferably 1 mass% or more, more preferably 2 mass% or more, and even more preferably 3 mass% or more from the viewpoint of wrinkle reducing effect and the like, and is preferably 20 mass% or less, more preferably 15 mass% or less, and even more preferably 10 mass% or less in the composition (B) from the viewpoint of wrinkle reducing effect and the like. Specifically, in the composition (B), 1

mass% or more and 20 mass% or less are preferable, 2 mass% or more and 15 mass% or less are more preferable, and 3 mass% or more and 10 mass% or less are even more preferable.

The content mass ratio of component (b3) to component (b2), [(b3)/(b2)], is preferably 0.05 or more, more preferably 0.1 or more, even more preferably 0.4 or more, from the viewpoint of wrinkle reducing effect, improving adhesion between skin and coating film, and the like, and is preferably 200 or less, more preferably 30 or less, and even more preferably 10 or less from the viewpoint of wrinkle reducing effect and the like. Specifically, 0.05 or more and 200 or less are preferable, 0.1 or more and 30 or less are more preferable, and 0.4 or more and 10 or less are even more preferable.

(Component (b4))

Examples of the component (b4) surfactant include a nonionic surfactant, an anionic surfactant, and an amphoteric surfactant, but a nonionic surfactant is preferable from the viewpoint of wrinkle reducing effect and the like. Note that examples of the anionic surfactant and the amphoteric surfactant include the same as those mentioned in Component (a1-3).

The HLB of the nonionic surfactant is preferably 3 or more and 20 or less, more preferably 4 or more and 18 or less, and even more preferably 4.5 or more and 15 or less.

Here, HLB (Hydrophilic-Lipophilic Balance) indicates the molecular weight of the hydrophilic group portion occupying the total molecular weight of the surfactant, and for the nonionic surfactant, it is obtained by the formula of Griffin.

The HLB of the mixed surfactant composed of 2 or more nonionic surfactants is obtained by additively averaging the HLB value of each nonionic surfactant based on its blending ratio.

$$\text{Mixture HLB} = \Sigma(\text{HLBx} \times \text{Wx})/\Sigma\text{Wx}$$

HLBx shows the HLB-value of the nonionic surfactant X.
Wx indicates mass (g) of the nonionic surfactant X with the value of HLBx.

Nonionic surfactant may be polyglycerin fatty acid ester, polyethylene glycol fatty acid ester, sucrose fatty acid ester, polyoxyethylene fatty acid ester, polyoxyethylene sorbitan fatty acid ester, polyoxyethylene alkyl ester, polyoxyethylene sorbitol fatty acid ester, polyoxyethylene glycol fatty acid ester, polyoxyethylene propylene glycol fatty acid ester, polyoxyethylene castor oil, polyoxyethylene cured castor oil, polyoxyethylene cured castor oil fatty acid ester, polyoxyethylene phytostanol ether, polyoxyethylene phytosterol ether, polyoxyethylene cholestanol ether, polyoxyethylene cholesteryl ether, polyoxyalkylene modified organopolysiloxane, polyoxyalkylene and alkyl co-modified organopolysiloxanes, sorbitan fatty acid esters, alkyl glyceryl ether and polyoxyalkylene-modified silicone.

Examples of the sorbitan fatty acid ester include sorbitan monopalmitate, sorbitan monostearate, sorbitan monooleate, sorbitan sesquioleate, sorbitan coconut oil fatty acid, sorbitan tristearate, and the like. Examples of the alkyl glyceryl ether include isostearyl glyceryl ether and the like. Examples of the polyoxyalkylene-modified silicone include a polyoxyethylene-methylpolysiloxane copolymer and a poly (oxyethylene-oxypropylene)-methylpolysiloxane copolymer.

The component (b4) may be used alone, or two or more kinds thereof may be used in combination.

The content of the component (b4) in the composition (B) is preferably 0.05 mass% or more, more preferably 0.1 mass% or more, and even more preferably 0.2 mass% or more from the viewpoint of wrinkle reducing effect and the like, and is preferably 16 mass% or less, more preferably 4 mass% or less, and even more preferably 2 mass% or less in the composition (B) from the viewpoint of wrinkle reducing effect and the like. Specifically, in the composition (B), 0.05 mass% or more and 16 mass% or less are preferable, 0.1 mass% or more and 4 mass% or less are more preferable, and 0.2 mass% or more and 2 mass% or less are even more preferable.

The content mass ratio as the component (b4) to component (b2), [(b4)/(b2)], is preferably 0.0025 or more, more preferably 0.006 or more, even more preferably 0.025 or more, from the viewpoint of wrinkle reducing effect and the like, and is preferably 160 or less, more preferably 8 or less, and even more preferably 2 or less from the viewpoint of wrinkle reducing effect and the like. Specifically, [(b4)/(b2)] is preferably 0.0025 or more and 160 or less, more preferably 0.006 or more and 8 or less, and even more preferably 0.025 or more and 2 or less.

(Component (b5))

The water-soluble thickener may be any one used in ordinary cosmetics, and examples thereof include carrageenan, dextrin, methylcellulose, ethylcellulose, hydroxyethylcellulose, hydroxypropylcellulose, polyvinyl alcohol, polyacrylic acid, polymethacrylic acid, carboxy vinyl polymer, acrylic acid/alkyl acrylate copolymer, xanthan gum, carboxy methylchitin, chitosan, and the like.

Among these, from the viewpoint of wrinkle reducing effect, stability, and feeling of use, a (meth) acrylic acid-based polymer is preferable, a carboxy vinyl polymer and an acrylic acid/alkyl acrylate copolymer are more preferable, and a (acrylic acid/alkyl acrylate (C10-30)) copolymer is particularly preferable.

Here, the (acrylic acid/alkyl acrylate (C10-30)) copolymer is obtained by crosslinking a copolymer of alkyl acrylate (C10-30), acrylic acid, and methacrylic acid or a lower alkyl ester thereof with an allyl ether of sucrose or an allyl ether of pentaerythritol, and a commercially available product such as Pemulen TR-1, Pemulen TR-2 (These are manufactured by Lubrizol) or the like can be used.

An acid-type water-soluble thickener such as a polyacrylic acid, a carboxy vinyl polymer, or a (acrylic acid/alkyl (C10-30) acrylate) copolymer may be used as a water-soluble or water-dispersible salt using an alkali metal hydroxide such as potassium hydroxide or sodium hydroxide as a neutralizing agent.

The component (b5) may be used alone, or in combination of two or more.

The content of the component (b5) in the composition (B) is preferably 0.05 mass% or more, more preferably 0.1 mass% or more, and even more preferably 0.2 mass% or more from the viewpoint of wrinkle reducing effect and the like, and is preferably 1 mass% or less, more preferably 0.8 mass% or less, and even more preferably 0.7 mass% or less in the composition (B) from the viewpoint of wrinkle reducing effect and the like. Specifically, the content of the component (b5) in the composition (B) is preferably 0.05 mass% or more and 1 mass% or less, more preferably 0.1 mass% or more and 0.8 mass% or less, and even more preferably 0.2 mass% or more and 0.7 mass% or less.

The content mass ratio of the component (b5) to the component (b2), [(b5)/(b2)], is preferably 0.0025 or more, more preferably 0.006 or more, even more preferably 0.025 or more, from the viewpoint of wrinkle reducing effect and the like, and is preferably 10 or less, more preferably 2 or less, and even more preferably 1 or less from the viewpoint of wrinkle reducing effect and the like. Specifically, 0.0025 or more and 10 or less are preferable, 0.006 or more and 2 or less are more preferable, and 0.025 or more and 1 or less are even more preferable.

(Component (b6))

The content of water in the composition (B) is preferably 55 mass% or more, more preferably 65 mass% or more, and even more preferably 70 mass% or more from the viewpoint of wrinkle reducing effect and the like, and is preferably 99 mass% or less, more preferably 95 mass% or less, and even more preferably 90 mass% or less in the composition (B) from the viewpoint of wrinkle reducing effect and the like. Specifically, in the composition (B), 55 mass% or more and 99 mass% or less are preferable, 65 mass% or more and 95 mass% or less are more preferable, and 70 mass% or more and 90 mass% or less are even more preferable.

The composition (B) may contain an active ingredient, an additive, and the like in addition to those commonly used in cosmetics. Examples of such an active ingredient or an additive include a water-soluble vitamin, a sequestering agent, ethanol, an antiseptic, an antioxidant, a dye, a pH adjusting agent, a perfume, an ultraviolet absorber, a humectant, a blood circulation accelerator, a cold feeling agent, an antiperspirant, a fungicide, a whitening agent, an anti-inflammatory agent, a skin activator, and the like may be contained. These may be used alone or in combination of two or more.

Here, the term "containing carbon dioxide gas" in the present invention means that carbon dioxide gas can be supplied to the skin, and for example, a liquid composition in which carbon dioxide gas is dissolved, a stock solution and a propellant are filled in a pressure-resistant container, and an aerosol composition containing carbon dioxide gas as a propellant (JP-A-2014-129306, JP-A-2017-125003, etc.), a foaming composition containing a carbon dioxide gas generating agent which reacts with water to generate carbon dioxide gas, a nonwoven fabric sheet containing the above-mentioned carbon dioxide gas generating agent (JP-A-2015-105451), and a pad containing the above-mentioned carbon dioxide gas generating agent (JP-A-2006-249025) are mentioned. Examples of the carbon dioxide gas generator include a combination of an acid and a carbonate salt.

Among such forms, from the viewpoint of wrinkle reducing effect, feeling of use, and the like, an aerosol composition in which a stock solution and a propellant are filled in a pressure-resistant container and contains carbon dioxide gas as a propellant is preferable. Such aerosol compositions include spray type, foam type, but foam type is preferable.

When the composition (B) is an aerosol composition, a mass ratio of the stock solution to the carbon dioxide gas (stock solution/carbon dioxide gas) is preferably 94/6 or more, more preferably 95/5 or more, particularly preferably 96.5/3.5 or more, and is also preferably 99.5/0.5 or less, more preferably 99/1 or less, particularly preferably 98.5/1.5 or less. Specifically, the mass ratio is preferably 94/6 or more and 99.5/0.5 or less, more preferably 95/5 or more and 99/1 or less, and particularly preferably 96.5/3.5 or more and 98.5/1.5 or less.

In addition to the carbon dioxide gas, the aerosol composition may contain a propellant used in an ordinary aerosol cosmetic. Examples of the propellant include liquefied petroleum gas and compressed gas. The content of carbon dioxide relative to the propellant total is preferably from 90 to 100 %, more preferably from 95 to 100 % by mass,

and particularly preferably from 98 to 100 % by mass.

The pH of the composition (B) (stock solution for an aerosol composition) at 25°C is preferably from 6.0 to 7.5, more preferably from 6.3 to 7.4, and particularly preferably from 6.5 to 7.3.

The viscosity of the composition (B) (stock solution for an aerosol composition) at 25°C, from the viewpoint of stability, discharge property, and the like, is preferably 500 mPa·s or more and 20 000 mPa·s or less, more preferably 1 000 mPa·s or more and 10 000 mPa·s or less, and particularly preferably 1 500 mPa·s or more and 7 000 mPa·s or less.

The above viscosity is a value measured by using a BM viscometer (manufactured by Toki Sangyo Co., Ltd.) at 25°C.

The composition (B) can be produced by an ordinary method. In the case of an aerosol composition, a stock solution may be prepared, and the obtained stock solution may be filled into a pressure-resistant container together with a propellant.

"Application" in the Step I (Step IA, Step IB) may be implemented by conventional coating film means according to the type of composition used in Step I (Step IA, Step IB), for example, fingers, palms, a means for spreading or restraining with a dedicated tool, and the like.

Step I is a step of applying one or two kinds of compositions selected from the composition (A) and the composition (B) to the skin, but in the Step I, both the composition (A) and the composition (B) are preferably applied from the viewpoint of wrinkle reducing effect and the like, and more preferably applied in the order of the composition (B) first and then the composition (A) .

The amount of the composition (A) to be used in the step IA is preferably 0.1 $mg/cm^2$ or more and 4 $mg/cm^2$ or less, and particularly preferably 0.2 $mg/cm^2$ or more and 3.5 $mg/cm^2$ or less.

The amount of the composition (B) to be used in the step IB is preferably 0.1 $mg/cm^2$ or more and 5.5 $mg/cm^2$ or less, and particularly preferably 0.2 $mg/cm^2$ or more and 5 $mg/cm^2$ or less.

When the step IA and the step IB are implemented, the total application amount of the composition (A) and the composition (B) is preferably 0.1 $mg/cm^2$ or more and 7 $mg/cm^2$ or less, and particularly preferably 0.2 $mg/cm^2$ or more and 6 $mg/cm^2$ or less.

-Step II-

Step II is a step of applying a coating film composed of a deposit containing a fiber to the skin.

Here, in the method for reducing wrinkles of the present invention, the step I and the step II are implemented in this order or in the reverse order, and a coating film composed of a deposit containing a fiber and one or two compositions selected from the group consisting of the composition (A) and the composition (B) may be applied so as to be laminated on the same site of the skin.

That is, implementing the step I and then applying a coating film composed of a deposit containing a fiber to the skin to which the composition is applied in the step I may be implemented, and then a step of applying one or two compositions selected from the group consisting of the composition (A) and the composition (B) to the skin to which a coating film composed of a deposit containing a fiber is applied in the step II may be implemented. When the step IA and the step IB are implemented, the order is not specifically limited-it may be in the order of step IA, step IB, and step II; in the order of step IA, step II, and step IB; in the order of the step IB, the step IA, and the step II; in the order of step IB, step II, and step IA; in the order of step II, step IA, and step IB; or in the order of step II, step IB, and step IA.

Among these methods for reducing wrinkles, from the viewpoint of wrinkle reducing effect and the like, it is preferable to implement the step I and then applying a coating film composed of a deposit containing a fiber to the skin to which the composition is applied in step I, and more preferably, the step IB, the step IA, and the step II are implemented in this order.

As long as the one or two compositions selected from the composition (A) and the composition (B) and the coating film composed of the deposit containing a fiber come into contact with each other on the skin (specifically, laminated), an additional cosmetic and a milky lotion may be further applied between the step I and the step II, or when the composition (A) and the composition (B) are used in combination, between the application of the composition (A) and the application of the composition (B). When the step II is implemented subsequently to the step I, a step of applying a liquid agent containing one or more selected from the group consisting of water, polyols and oils in a liquid state at 25°C to the skin to which the coating film is applied in the step II by a method other than an electrostatic spraying may be implemented.

In the method for producing a coating film according to the present invention, the step IB and the step II may be implemented in this order or in the reverse order, and the composition (B) and the coating film composed of a deposit containing a fiber may be applied so as to be laminated on the same site of the skin.

That is, implementing the step IB and then applying a coating film composed of a deposit containing a fiber on the skin to which the composition (B) is applied in the step IB may be implemented, or a step of applying the composition (B) on the skin to which a coating film composed of a deposit containing a fiber is applied in the step II and then a step

of applying the composition (B) may be implemented in the step II

As a method for producing a coating film of the present invention, it is preferable to further include a step IA in addition to the step IB and the step II, from the viewpoint of wrinkle reducing effect, or the like. When the method involves the step IA, the method is not specifically limited. The method may be implemented in an order of the step IA, the step IB; and the Step II; an order of the step IA, the step II, and the step IB; an order of the step IB, the step IA, and the step II; an order of the step IB, the step II, the step IA; an order of the step II, the step IA, and the step IB; an order of the step II, the step IB, and the step IA.

Among these methods for producing a coating film, from the viewpoint of wrinkle reducing effect or the like, it is preferable to implement the step IB and then applying a coating film composed of a deposit containing a fiber to the skin to which the composition (B) was applied to in the step IB. It is more preferably to implement the step IB, the step IA, and the step II in this order.

As long as the composition (B) and the coating film composed of a deposit containing a fiber come into contact with each other on the skin (specifically, laminated), an additional cosmetic composition or emulsion may be applied between the step IB and the step II, or between the application of the composition (A) and the application of the composition (B) where the composition (A) and the composition (B) are used in combination. When the step II is implemented next to the step IB, a step of applying a liquid agent containing one or more kinds selected from water, a polyol or an oil liquid at 25°C to the skin to which the coating film is applied to in the step II by means other than electrostatic spraying may be implemented.

The coating film composed of a deposit containing a fiber is preferably a coating film formed by electrostatically spraying a composition X containing the following component (c1) and the component (c2), and more preferably a coating film formed by electrospinning the composition X. The deposit may further contain a material other than fibers (e.g., a liquid material present around the fibers). The coating film composed of a deposit containing a fiber is preferably a coating film composed of a deposit containing a fiber by the component (c2). For example, when a water-insoluble film-forming polymer is used as the component (c2), a coating film composed of a deposit containing a fiber of a water-insoluble polymer may be obtained.

(c1) one or more volatile substances selected from water, alcohols and ketones
(c2) a film-forming polymer

The composition X is a composition for forming a coating film by electrostatic spraying (spraying composition).

The (c1) volatile substance is a substance exhibiting volatility in its liquid state. In the composition X, the component (c1) is sufficiently electrically charged when placed in an electric field, and is discharged from the tip of the nozzle toward the object to be sprayed, and when the component (c1) evaporates, the charge density of the composition X becomes excessive, and the component (c1) further evaporates while being refined by Coulomb repulsion, and is finally blended for the purpose of forming a dry coating film. For this purpose, the (c1) volatile substance preferably has a vapor pressure at 20°C of 0.01 kPa or more and 106.66 kPa or less, more preferably 0.13 kPa or more and 66.66 kPa or less, even more preferably 0.67 kPa or more and 40.00 kPa or less, further more preferably 1.33 kPa or more and 40.00 kPa or less, and particularly preferably 2.40 kPa or more and 40.00 kPa or less.

Among the (c1) volatile substance, examples of the alcohol include a monohydric chain aliphatic alcohol, a monohydric cyclic aliphatic alcohol, and a monohydric aromatic alcohol.

Examples of the monohydric chain aliphatic alcohol include a linear or branched chain C1-6 alcohol. Examples of the monohydric cyclic aliphatic alcohol include a cyclic aliphatic C4-6 alcohol. Examples of the monohydric aromatic alcohol include benzyl alcohol, phenylethyl alcohol, and the like. Specifically, methanol, ethanol, isopropyl alcohol, n-propyl alcohol, n-butyl alcohol, 2-butyl alcohol, isobutyl alcohol, 2-methyl-2-propyl alcohol, n-pentanol, 2-pentanol, 3-pentanol, 2-methyl-1-butyl alcohol, 2-methyl-2-butyl alcohol, 3-methyl-1-butyl alcohol, 3-methyl-2-butyl alcohol, neopentyl alcohol, n-hexanol, 2-hexanol, 3-hexanol, 2-methyl-1-pentanol, 3-methyl-1-pentanol, 4-methyl-1-pentanol, 2-methyl-2-pentanol, 3-methyl-2-pentanol, 4-methyl-2-pentanol, 2-methyl-3-pentanol, 3-methyl-3-pentanol, 2,2-dimethyl-1-butanol, 2,3-dimethyl-1-butanol, 3,3-dimethyl-1-butanol, 2,3-dimethyl-2-butanol, 3,3-dimethyl-2-butanol, 2-ethyl-1-butanol, cyclobutanol, cyclopentanol, cyclohexanol, benzyl alcohol, and phenylethyl alcohol. These alcohols may be used alone or in combination of two or more selected from these.

Among the (c1) volatile substance, examples of ketones include ketones having two C1-4 alkyl groups, such as acetone, methyl ethyl ketone, and methyl isobutyl ketone. These ketones may be used alone or in combination of two or more.

The component (c1) is preferably water, alcohol or a mixture thereof from the viewpoint of suppressing clogging of the nozzle, and from the viewpoint of fiber formability, one or more selected from the group consisting of water, ethanol, isopropyl alcohol and n-butyl alcohol are more preferable, and a combination of water and one or more selected from the group consisting of ethanol, isopropyl alcohol and n-butyl alcohol is even more preferable. A combination of water and ethanol is particularly preferable. The content of water in the component (c1) is preferably 0.1 mass% or more and

15 mass% or less, and more preferably 0.2 mass% or more and 5 mass% or less, from the viewpoint of fiber formability.

The component (c1) may be used alone, or two or more kinds thereof may be used in combination.

The content of the component (c1) in the composition X is preferably 45 mass% or more, more preferably 50 mass% or more, even more preferably 55 mass% or more, particularly preferably 60 mass% or more, and also preferably 95 mass% or less, more preferably 93 mass% or less, even more preferably 90 mass% or less, particularly preferably 88 mass% or less from the viewpoint of fiber formability. From the same viewpoint, the content of the component (c1) in the composition X is preferably 45 mass% or more and 95 mass% or less, more preferably 50 mass% or more and 93 mass% or less, even more preferably 55 mass% or more and 90 mass% or less, and particularly preferably 60 mass% or more and 88 mass% or less. By containing the component (c1) in the composition X at this ratio, a target coating film can be efficiently formed, and a coating film formed of a fiber can be stably formed. By containing the component (c1) in the composition X at this ratio, it is possible to efficiently and sufficiently volatilize the component (c1) from the composition X when the electrostatic spraying method is implemented.

The (c2) film-forming polymer is generally a substance dissolved in the (c1) volatile substance. Here, "dissolved" represents that when the component (c1) and the component (c2) are mixed, the component (c2) is in a dispersed state in the component (c1) at 20°C, and the dispersion state is a visually uniform state, preferably a visually transparent or translucent state. The film-forming ability in the present invention is preferably a fiber-forming ability.

The film-forming polymer may be appropriately chosen in accordance with the property of the (c1) volatile substance. Specifically, a film-forming polymer is roughly classified into a water-soluble polymer and a water-insoluble polymer. As used herein, the term "water-soluble polymer" means a polymer having a property in which, after weighing the polymer 1 g at 1 atm and 23°C, the polymer is immersed in 10g of ion-exchanged water, and 0.5 g or more of the immersed polymer is dissolved in water after a lapse of 24 hours. In the present specification, the "water-insoluble polymer" refers to a polymer having a property in which 1 g of the polymer is weighed and then immersed in 10 g of ion-exchanged water in an environment at 1 atm and 23°C, and after a lapse of 24 hours, only less than 0.5 g of the immersed polymer is dissolved in water.

Examples of the water-soluble film-forming polymer include a mucopolysaccharide such as pullulan, hyaluronic acid, chondroitin sulfate, poly-γ-glutamic acid, modified corn starch, β-glucan, a gluco-oligosaccharide, heparin, and keratosulfuric acid; a natural polymer such as cellulose, pectin, xylan, lignin, glucomannan, galacturonic acid, thyrium seed gum, tamarind seed gum, gum arabic, gum tragacanth, a soy water-soluble polysaccharide, alginic acid, carrageenan, laminan, agar (agarose), fucoidan, methylcellulose, hydroxypropyl cellulose, and hydroxypropyl methyl cellulose; a synthetic polymer such as a partially saponified polyvinyl alcohol (not used in combination with a crosslinking agent), a low saponified polyvinyl alcohol, polyvinyl pyrrolidone (PVP), polyethylene oxide, and sodium polyacrylate. These water-soluble polymers may be used alone or in combination of two or more. Among these water-soluble polymers, pullulan, and synthetic polymers such as partially saponified polyvinyl alcohol, low saponified polyvinyl alcohol, polyvinyl pyrrolidone, and polyethylene oxide are preferably used from the viewpoint of easy manufacturing of the coating film. When polyethylene oxide is used as the water-soluble polymer, its number average molecular weight is preferably 50 000 or more and 3 000 000 or less, more preferably 100 000 or more and 2 500 000 or less.

Meanwhile, examples of the water-insoluble film-forming polymer include, a fully saponified polyvinyl alcohol which can be insolubilized after the formation of a coating film, a partially saponified polyvinyl alcohol which can be crosslinked after the formation of a coating film by using in combination with a crosslinking agent, an oxazoline-modified silicone such as a poly(N-propanoylethyleneimine)-dimethylsiloxane/γ-aminopropylmethylsiloxane copolymer, polyvinyl acetal diethyleminoacetate, Zein (a main component of corn protein), polyester resin such as polylactic acid (PLA), an acrylic resin (e.g., a polyacrylonitrile resin, a polymethacrylic acid resin), a polystyrene resin, a polyvinyl butyral resin, a polyethylene terephthalate resin, a polybutylene terephthalate resin, a polyurethane resin, a polyamide resin, a polyimide resin, a polyamide-imide resin. These water-insoluble polymers can be used alone or in combination of two or more.

Among these water-insoluble polymers, it is preferable to use one or more selected from the group consisting of a fully saponified polyvinyl alcohol which can be insolubilized after the formation of a coating film, a partially saponified polyvinyl alcohol which can be crosslinked after the formation of a coating film by using in combination with a crosslinking agent, a polyvinyl butyral resin, a polyurethane resin, an oxazoline-modified silicone such as a poly(N-propanoylethyleneimine) graft-dimethylsiloxane/γ-aminopropylmethylsiloxane copolymer, polyvinyl acetal diethylaminoacetate and Zein.

The content of the component (c2) in the composition X is preferably 4 mass% or more, more preferably 6 mass% or more, and further more preferably 8 mass% or more. The content of the component (c2) in the composition X is preferably 35 mass% or less, more preferably 30 mass% or less, further more preferably 25 mass% or less, and even more preferably 20 mass% or less. The content of the component (c2) in the composition X is preferably 4 mass% or more and 35 mass% or less, more preferably 4 mass% or more and 30 mass% or less, even more preferably 6 mass% or more and 25 mass% or less, and particularly preferably 8 mass% or more and 20 mass% or less. The ratio of the component (c2) in the composition X serves to efficiently form a target coating film, and to stably form a coating film formed of fibers.

The mass ratio of the content of the component (c1) and the component (c2) in the composition X, ((c1)/(c2)), is preferably 0.5 or more and 40 or less, more preferably 1 or more and 30 or less, and further more preferably 2 or more and 25 or less, from the viewpoint of sufficiently volatilizing the component (c1) when the electrostatic spraying method is implemented.

The mass ratio of the content of ethanol and the component (c2) in the composition X, ((c1)/(c2)), is preferably 0.5 or more and 40 or less, more preferably 1 or more and 30 or less, and even more preferably 2 or more and 25 or less, from the viewpoint of sufficiently volatilizing the component (c1) when the electrostatic spraying method is implemented.

The composition X is preferably, in addition to the component (c1) and the component (c2), those containing one or more selected from the group consisting of the (c3) glycols and the (c4) conductivity controlling agents.

Examples of the glycols include ethylene glycol, propylene glycol, butylene glycol, diethylene glycol, dipropylene glycol, polypropylene glycol, and the like.

The content of the component (c3) in the composition X is preferably 0.001 mass% or more and 10 mass% or less, and more preferably 0.01 mass% or more and 5 mass% or less.

Examples of the conductivity controlling agent include an alkali metal salt and an ammonium salt.

The content of the component (c4) in the composition X is preferably 0.01 mass% or more and 10 mass% or less, more preferably 0.05 mass% or more and 6 mass% or less, and even more preferably 0.1 mass% or more and 2.5 mass% or less.

In addition to the aforementioned components, the composition X may contain, for example, an oil agent such as di (phytosteryl/octyldodecyl)lauroyl glutamate, a powder, a UV protective agent, a perfume, a repellent, an antioxidant, a stabilizer, a preservative, an antiperspirant, various vitamins. These may be used alone, or two or more may be used in combination. The content of these other components is preferably 20 mass% or less, more preferably 10 mass% or less in the composition X.

Here, in this specification, "applying a coating film formed by electrostatic spraying to the skin" includes a mode in which the composition X is electrostatically sprayed onto a base to form a coating film in advance, and the obtained coating film (a sheet such as a film or a patch) is adhered to the skin, and a mode in which a coating film is formed by fibers directly on the skin by electrostatic spraying.

When the electrostatic spraying is implemented, a composition having a viscosity at 25°C of 1 mPa·s or more, more preferably 10 mPa·s or more, even more preferably 50 mPa·s or more is used as the spraying composition. A composition having a viscosity at 25°C of 2 000 mPa·s or less, more preferably 1 500 mPa·s or less, and even more preferably 1 200 mPa·s or less is used. The viscosity of the spraying composition at 25°C is preferably 1 mPa·s or more and 2 000 mPa·s or less, more preferably 10 mPa.s or more and 1 500 mPa.s or less, and even more preferably 50 mPa.s or more and 1 200 mPa.s or less. By using a spraying composition having a viscosity in this range, it is possible to successfully form a porous coating film, in particular a porous coating film composed of a fiber deposit, by means of an electrostatic spraying. The formation of the porous coating film is advantageous from the viewpoint of enhancing the stuffiness prevention, and adhesiveness of the coating film, and from the viewpoint of easily and finely removing the coating film from the skin. The viscosity of the spraying composition is measured at 25°C using a type-E viscometer. As the type-E viscometer, for example, an E-type viscometer manufactured by Tokyo Instrument Co., Ltd. can be used. In this case, the rotor No. 43 can be used as the rotor.

The composition X is sprayed directly onto the base or human skin by an electrostatic spraying method. The electrostatic spraying method includes the step of electrostatically spraying the composition X on the base or on the skin using an electrostatic spraying device in an electrostatic spray step to form a coating film. The electrostatic spraying device includes a container containing a composition X, a nozzle for discharging the composition X, a supply device for supplying the composition X contained in the container to the nozzle, and a power supply for applying a voltage to the nozzle. Fig. 1 is a schematic diagram showing a configuration of an electrostatic spraying device preferably used in the present invention. The electrostatic spraying device 10 shown in the figure includes a low-voltage power supply 11. The low-voltage power supply 11 can generate a voltage of several volts ranging from a few volts to just over dozen volts. For the purpose of enhancing the portability of the electrostatic spraying device 10, the low-voltage power supply 11 preferably comprises one or more batteries. By using a battery as the low-voltage power supply 11, there is an advantage that replacement can be easily implemented as necessary. Instead of the battery, an AC adapter or the like can be used as the low-voltage power supply 11.

The electrostatic spraying device 10 also includes a high-voltage power supply 12. The high-voltage power supply 12 is connected to the low-voltage power supply 11, and includes an electric circuit (not shown) for boosting a voltage generated by the low-voltage power supply 11 to a high voltage. The booster electric circuit is generally composed of a transformer, a capacitor, a semiconductor element, and the like.

The electrostatic spraying device 10 further includes an auxiliary electrical circuit 13. The auxiliary electric circuit 13 is interposed between the low-voltage power supply 11 and the high-voltage power supply 12, and has a function of adjusting the voltage of the low-voltage power supply 11 to stably operate the high-voltage power supply 12. The auxiliary

electric circuit 13 has a function of controlling the number of revolutions of a motor provided in a microgear pump 14, which will be described later. By controlling the rotation speed of the motor, the supply amount of the spraying composition from the container 15 of the spraying composition described later to the microgear pump 14 is controlled. A switch SW is mounted between the auxiliary electric circuit 13 and the low-voltage power supply 11 so that the electrostatic spraying device 10 can be turned on and off by turning the switch SW on and off.

The supply amount of the composition X may be controlled by a pump for driving a piston in which, in addition to the microgear pump 14, the container 15 containing the composition X has, for example, a cylindrical side wall and slides inside the side wall to reduce the volume.

The electrostatic spraying device 10 further includes a nozzle 16. The nozzle 16 is made of a conductive material such as metal or a non-conductive material such as plastic, rubber, ceramic, or the like, and has a shape capable of discharging the spraying composition from the tip thereof. A minute space through which the spraying composition flows is formed in the nozzle 16 along the longitudinal direction of the nozzle 16. The size of the cross section of the micro space is preferably 100 um or more and 1 000 um or less in terms of diameter. The nozzle 16 communicates with the microgear pump 14 via a pipe 17. The pipe 17 may be conductive or non-conductive. The nozzle 16 is electrically connected to the high-voltage power supply 12. This makes it possible to apply a high voltage to the nozzle 16. In this case, in order to prevent an excessive current from flowing when a human body directly touches the nozzle 16, the nozzle 16 and the high-voltage power supply 12 are electrically connected via a current limiting resistor 19.

The microgear pump 14, which communicates with the nozzle 16 via the pipe 17, functions as a supply device for supplying the nozzle 16 with the composition X contained in the container 15. The microgear pump 14 is operated by receiving a power supply from the low-voltage power supply 11. The microgear pump 14 is configured to supply a predetermined amount of the composition X to the nozzle 16 under the control of the auxiliary electric circuit 13.

The container 15 is connected to the microgear pump 14 via a flexible pipe 18. Contained in the container 15 is the composition X. The container 15 preferably has a cartridge-type replaceable configuration.

The electrostatic spraying device 10 having the above configuration can be used, for example, as shown in Fig. 2. Fig. 2 shows an electrostatic spraying device 10 of the hand-held type having a size holdable by one hand. The electrostatic spraying device 10 shown in the same figure contains all of the members of the configuration diagram shown in Fig. 1 in the cylindrical housing 20. A nozzle, not shown, is disposed at one longitudinal end 10a of the housing 20. The nozzle is disposed in the housing 20 such that the blowing direction of the composition coincides with the longitudinal direction of the housing 20 and is convex toward the skin side. By arranging the nozzle tip so as to be convex toward the skin in the longitudinal direction of the housing 20, the spraying composition hardly adheres to the housing, and the coating film can be stably formed.

When the skin to be coated is the user's own skin, in order to operate the electrostatic spraying apparatus 10, the user, i.e. the person who forms the coating film on his or her own skin by the electrostatic spraying method, grasps the device 10 by his or her hand and directs one end 10a of the device 10, in which a nozzle (not shown) is located, to the site to be sprayed electrostatically. In Fig. 2, one end 10a of the electrostatic spraying device 10 is shown pointing inside the user's forearm. In this state, the device 10 is switched on to implement the electrostatic spraying method. When the device 10 is powered on, an electric field is generated between the nozzle and the skin. In the embodiment shown in Fig. 2, a positive high voltage is applied to the nozzle and the skin functions as a negative electrode. Since an electric field is generated between the nozzle and the skin, the composition X at the tip of the nozzle is polarized by electrostatic induction, so that the tip becomes a cone-like shape, and droplets of the composition X electrically charged from the tip of the cone are ejected into the air along the electric field toward the skin. As the component (c1) as a solvent evaporates from the composition X discharged into the space and electrically charged, the charge density on the surface of the composition X becomes excessive, and spreads into the space while repeatedly refining by the Coulombic repulsive force, and reaches the skin. In this case, the sprayed composition X can be delivered to the skin in the form of droplets by appropriately adjusting the viscosity of the composition X. Alternatively, while being discharged into the space, the (c1) volatile substance as a solvent is volatilized from the composition X, a film-forming polymer as a solute is solidified, and a fiber is formed with elongation and/or deformation by a potential difference, so that the fiber can be deposited on the skin surface. For example, elevated viscosity of the composition X can tend to cause the composition X to be deposited on the skin surface in the form of fiber. As a result, a coating film composed of a fiber deposit is formed on the skin surface. The coating film can also be formed by adjusting the distance between the nozzle and the skin, and/or the voltage applied to the nozzle.

During the electrostatic spraying method, a high potential difference is generated between the skin, which is the object to be coated, and the nozzle. However, because of the very large impedance, the current flowing through the human body is extremely small. For example, the present inventors confirmed that the current flowing through the human body during the electrostatic spraying method is several orders of magnitude smaller than the current flowing through the human body due to static electricity generated during normal human body.

When a deposit containing a fiber is formed by the electrostatic spraying method, the thickness of the fibers is preferably 10nm or more, more preferably 50 nm or more, when expressed as a circle equivalent diameter. It is preferably

3 000 nm or less, more preferably 2 000 nm or less, and even more preferably 1 000 nm or less. The thickness of the fiber can be measured by observing the fiber at a magnification of 10 000× by, for example, scanning electron microscopy (SEM) observation, removing defects (clumps of fibers, crossing portions of fibers, and droplets) from the two-dimensional image, arbitrarily selecting 10 fibers, drawing a line orthogonal to the longitudinal direction of the fiber, and directly reading the fiber diameter.

Such aforementioned fibers are continuous fibers of infinite length in principle of manufacture, but preferably have a length of at least 100 times or more the thickness of the fibers. In the present specification, a fiber having a length of 100 times or more of the thickness of the fiber is defined as a "continuous fiber". The coating film produced by the electrostatic spraying method is preferably a porous discontinuous coating film composed of a deposit of continuous fibers. Such a form of coating film not only can be handled as a single sheet as an aggregate, but also has an advantage in excellent softness, and integration hard to be separated even with a shearing force, and excellent followability to the body movement. It also has an advantage in excellent sweat dissipation generated out of the skin. It also has an advantage of easy peelability/removability.

The composition X reaches the skin in an electrically charged state. Since the skin is also electrically charged as described above, the fiber adheres to the skin by electrostatic forces. Since fine irregularities such as skin texture are formed on the skin surface, it is considered that the fibers are more closely adhered to the skin surface together with the anchor effect due to the unevenness. When the electrostatic spraying is completed in this manner, the power of the electrostatic spraying device 10 is turned off. As a result, the electric field between the nozzle and the skin disappears, and the skin surface is immobilized with an electric charge. As a result, the adhesion of the coating film is further developed.

Although the above description has been directed to a porous coating film composed of a deposit containing a fiber as a coating film, the form of the coating film is not limited to this, and a continuous coating film free of pores may be formed, or a porous coating film having a form other than the fiber deposit, for example, a porous coating film in which multiple through-holes are formed irregularly or regularly in the continuous coating film, i.e., a discontinuous coating film may be formed. As described above, a coating film of any shape can be formed by controlling the viscosity of the composition X, the distance between the nozzle and the skin, the voltage applied to the nozzle, and the like.

The distance between the nozzle and the skin also depends on the voltage applied to the nozzle, and a distance of 50 mm or more and 150 mm or less is preferable for successfully forming the coating film. The distance between the nozzle and the skin can be measured by a commonly used non-contact sensor or the like.

Whether or not the coating film formed by the electrostatic spraying method is porous, the basis weight of the coating film is preferably equal to or higher than 0.1 $g/m^2$, and more preferably equal to or higher than 1 $g/m^2$. It is preferably equal to or less than 40 $g/m^2$, more preferably equal to or less than 30 $g/m^2$. Specifically, the basis weight of the coating film is preferably 0.1 $g/m^2$ or more and 40 $g/m^2$ or less, more preferably 1 $g/m^2$ or more and 30 $g/m^2$ or less. By adjusting the basis weight of the coating film in this manner, adhesion of the coating film can be elevated. The electrostatic spraying step of directly electrostatically spraying the composition to the skin described herein to form a coating film denotes a step of electrostatically spraying onto the skin to form a coating film.

When a coating film formed by electrostatically spraying a composition X on a base in advance is applied to the skin, a composition X is electrostatically sprayed onto the base to prepare a sheet composed of a deposit containing a fiber, and the sheet is applied to the skin. Here, examples of the base include a resin and a metal plate-like base, a cosmetic puff, and a hand. The electrostatic spraying method is similar to as described above. It is preferable to trim a sheet made of a deposit containing the obtained fibers into a shape of a site to be applied, for example, it is preferable to trim the sheet into the shape covering only the area under the eye and the eye end, and to patch the sheet to the aforementioned area.

The holding time of the coating film composed of a deposit containing a fiber is preferably 4 to 20 hours, and from the viewpoint of wrinkle reducing effect, more preferably 6 to 12 hours, and even more preferably 8 to 10 hours.

While the method for reducing wrinkles and the method for producing a coating film have been described based on preferable embodiments, the present invention is not limited to the above embodiments. For example, in the embodiments described above, a person who intends to form a coating film on his or her own skin grips the electrostatic spraying device 10 and generates an electric field between the nozzle of the device 10 and his or her skin, but as long as an electric field is generated therebetween, it is not necessary for a person who intends to form a coating film on his or her own skin to grip the electrostatic spraying device 10.

The method for reducing wrinkles according to the present invention is excellent in the effect of reducing wrinkles on the skin. The method for producing a coating film according to the present invention facilitates forming a coating film on the skin that is excellent in wrinkle reducing effect of the skin.

Here, the "skin" on which the coating film is formed is preferably a skin of one or more sites selected from the group consisting of a face, a neck, a hand and a foot, more preferably a skin of a face, and even more preferably a skin of one or more selected from the group consisting of an outer end of the eye, a lower part from the eye, a mouth, a forehead, an eyelid and glabella, and particularly preferably a skin of one or more selected from the group consisting of an outer end of the eye and a lower part from the eye. The method for reducing wrinkles and the method for producing

a coating film according to the present invention are suitable for application on a wrinkle site of the skin, more suitable for application on a wrinkle site of a facial skin, and particularly suitable for application on a wrinkle site of a skin at one or more sites selected from the group consisting of an outer end of the eye and a lower part from the eye.

Here, examples of the "wrinkles" include large wrinkles (coarse lines), small wrinkles (fine lines), and the like. Large wrinkles refer to deep wrinkles which can be visually confirmed even when the skin is pulled. Small wrinkles refer to shallow and superficial wrinkles that cannot be visually confirmed when the skin is pulled. For both of these wrinkles, the present invention has a reduction effect. For example, the present invention is effective for reducing wrinkles of 0.15 to 0.47 mm thick and is particularly effective for reducing wrinkles of characterized with the thickness ranging from 0.28 to 0.47 mm. When the area indicated by the total of the lines of wrinkles to be detected becomes small or wrinkles become inconspicuous, it can be said that wrinkles are reduced, and it is preferable to reduce the wrinkle area by 10 to 30%, and it is more preferable to reduce the wrinkle area by 15 to 25%. In addition, it is effective in reducing wrinkles of grades 1 to 7 described in the Guideline for Evaluating AntiWrinkle Function (Journal of Japanese Cosmetic Science Society Vol. 30, No. 4, 316-332 (2006)), especially 3 to 5 thereof. When the coating film composed of a deposit containing a fiber is held for from 4 to 20 hours (preferably from 6 to 12 hours, more preferably from 8 to 10 hours) per day and then peeled off, it is preferably repeated for from 3 to 24 weeks, more preferably from 3 to 16 weeks, and particularly preferably from 3 to 6 weeks.

[Kit for film formation, coating film]

A kit for forming a coating film according to the present invention may include the above composition (B) and a coating film composed of a deposit containing a fiber or a composition for forming the coating film.

The kit for forming a coating film according to the present invention is preferably a kit further including the above composition (A) from the viewpoint of reducing wrinkles. When the above composition for forming a coating film (e.g., composition X described above) is provided, it is preferable that the kit for forming a coating film according to the present invention further includes the above-described electrostatic spraying device. The coating film composed of a deposit containing a fiber provided in the kit for forming a coating film is preferably a sheet formed of a deposit containing a fiber described above.

The coating film for application to the skin according to the present invention contains a deposit containing a fiber and carbon dioxide gas. The coating film for application to the skin according to the present invention preferably further contains an amphiphilic molecule from the viewpoint of wrinkle reducing effect. The coating film may contain each components of the compositions (A) and (B).

The meaning of terms in the kit for forming a coating film, and the coating film, a content of each component, a ratio thereof, and the like are the same as those described for the method for reducing wrinkles, and the method for producing a coating film according to the present invention. The coating film according to the present invention preferably contains carbon dioxide gas in one or two selected from a space between fibers and the fiber surface, and more preferably contains one or two selected from an amphiphilic molecule and carbon dioxide gas in the above one or two selected from a space between fibers and the fiber surface.

In the coating film for application to the skin according to the present invention, the content mass ratio of the carbon dioxide per $m^2$ of the deposit containing a fiber ($\gamma$) to the fiber per $m^2$ of the deposit containing a fiber ($\alpha$), ($\gamma$)/($\alpha$), is preferably 1 or more, more preferably 5 or more, even more preferably 10 or more, particularly preferably 15 or more, from the viewpoint of improving adhesion between the skin and the coating film, and the like, and is preferably 45 or less, more preferably 40 or less, even more preferably 35 or less, particularly preferably 30 or less from the viewpoint of improving adhesion between the skin and the coating film, and the like. Specifically, ($\gamma$)/($\alpha$) is preferably 1 or more and 45 or less, more preferably 5 or more and 40 or less, even more preferably 10 or more and 35 or less, and particularly preferably 15 or more and 30 or less.

In the coating film for application to the skin according to the present invention, the content mass ratio of the amphiphilic molecule per $m^2$ of the deposit containing a fiber ($\beta$) to the fiber per $m^2$ of the deposit containing a fiber ($\alpha$), ($\beta$)/($\alpha$), is preferably 0.1 or more, more preferably 0.5 or more, even more preferably 1 or more, particularly preferably 2 or more, from the viewpoint of improving the adhesion between the skin and the coating, and from the viewpoint of improving the transparency, and the like, preferably 30 or less, more preferably 20 or less, even more preferably 17 or less, and particularly preferably 15 or less from the viewpoint of improving the adhesion between the skin and the coating film, and improving the transparency. Specifically, ($\beta$)/($\alpha$) is preferably 0.1 or more and 30 or less, more preferably 0.5 or more and 20 or less, even more preferably 1 or more and 17 or less, and particularly preferably 2 or more and 15 or less.

In this specification, mass of fibers per $m^2$ of deposit containing a fiber means (c2) mass of the film-forming polymer.

The coating film for application to the skin according to the present invention can be produced by a method for producing a coating film according to the present invention or by using a kit for forming a coating film according to the present invention.

In the present specification, use for reducing wrinkles on skin is preferably non-therapeutic use such as use for cosmetic purposes. "Non-therapeutic" refers to a concept that does not include medical practice, i.e., does not include a method of operating, treating or diagnosing humans, more specifically, does not include a method of implementing surgery, therapy or diagnosis on humans by a physician, or a medical practitioner or physician directed.

In relation to the above embodiments, the present invention further discloses the following embodiments.

<1> A method for producing a coating film on skin, the method comprising

(Step IB) applying the following composition (B) onto the skin and
(Step II) applying a coating film composed of a deposit containing a fiber to the skin;
in this order or in the reverse order:
Composition (B): a composition comprising carbon dioxide gas.

<2> A method for producing a coating film on skin, the method comprising

(Step IB) applying the following composition (B) to the skin and
(Step II) applying a coating film composed of a deposit containing a fiber to the skin to which the composition (B) was applied in the step IB;
Composition (B): a composition comprising carbon dioxide gas.

<3> The method for producing a coating film on skin according to <1> or <2>, further comprising

(Step IA) applying the following composition (A) onto the skin;
Composition (A): a composition comprising an amphiphilic molecule and having a lamellar structure.

<4> The method for producing a coating film on skin according to <3>, wherein the amphiphilic molecule is selected from the group consisting of (a1-1) ceramides, (a1-2) polyol fatty acid esters having an intramolecular hydroxy group, (a1-3) surfactants and (a1-4) higher alcohols.
<5> The method for producing a coating film on skin according to <4>, wherein the component (a1-2) is one or more selected from the group consisting of glycerol monofatty acid esters, glycerol difatty acid esters, sorbitan monofatty acid esters, sorbitan difatty acid esters and diglycerol difatty acid esters.
<6> The method for producing a coating film on skin according to <4> or <5>, wherein the component (a1-3) is an ionic surfactant.
<7> The method for producing a coating film on skin according to any one of <4> to <6>, wherein the component (a1-4) is a monovalent C10-24 alcohol.
<8> The method for producing a coating film on skin according to any one of <3> to <7>, wherein the composition (A) further comprises one or more selected from (a2) an oily component other than the component (a1), (a3) a polyol and (a4) water.
<9> The method for producing a coating film on a skin according to any one of <1> to <8>, wherein the composition (B) further comprises one or more selected from the group consisting of (b2) oil agents, (b3) polyols, (b4) surfactants, (b5) water-soluble thickeners and (b6) water.
<10> The method for producing a coating film on skin according to <9>, wherein the component (b4) is a nonionic surfactant having a HLB value of from 3 to 20.
<11> The method for producing a coating film on skin according to <9> or <10>, wherein the component (b5) is a (meth) acrylic acid-based polymer.
<12> The method for producing a coating film on skin according to any one of <1> to <11>, wherein the composition (B) is an aerosol composition in which a stock solution and a propellant are filled in a pressure-resistant container and contains carbon dioxide gas as a propellant.
<13> The method for producing a coating film on skin according to any one of <1> to <12>, wherein a coating film composed of a deposit containing a fiber is a coating film formed by electrostatically spraying a composition X comprising the following components (c1) and (c2):

(c1) one or more volatile substances selected from the group consisting of water, alcohols and ketones;
(c2) a film-forming polymer.

<14> The method for producing a coating film on skin according to any one of <1> to <13>, wherein the coating film composed of a deposit containing a fiber is a coating film composed of a deposit containing a fiber of a water-insoluble polymer.

<15> The method for producing a coating film on skin according to any one of <1> to <14>, wherein a thickness of the fiber is from 10 nm to 3 000 nm with a circle equivalent diameter.
<16> The method for producing a coating film on skin according to any one of <1> to <15>, wherein the step II is a step of sticking a coating sheet made of a deposit containing a fiber to the skin, or a step of forming a coating film composed of a deposit containing a fiber directly on the skin by electrostatic spraying.
<17> The method for producing a coating film on skin according to any one of <1> to <16>, on skin of a face.
<18> The method for producing a coating film on skin according to any one of <1> to <17>, on a wrinkle site of skin.
<19> A coating film for application to skin comprising a deposit containing a fiber, and carbon dioxide gas.
<20> The coating film according to <19>, further comprising an amphiphilic molecule.
<21> The coating film according to <20>, wherein one or two of components selected from amphiphilic molecules and carbon dioxide gas are contained in one or two selected from the group consisting of a space between fibers and the surface of the fiber.
<22> The coating film according to <20> or <21>, wherein the content mass ratio of mass ($\beta$) of the amphiphilic molecule per $m^2$ of the deposit containing a fiber to mass ($\alpha$) of the fiber per $m^2$ a deposit containing a fiber, $[(\beta)/(\alpha)]$, is 0.1 or more and 30 or less.
<23> The coating film according to any one of <19> to <22>, wherein the deposit containing a fiber is a deposit containing a fiber of a water-insoluble polymer.
<24> The coating film according to any one of <19> to <23>, wherein a thickness of the fiber is from 10 nm to 3 000 nm in a circle equivalent diameter.
<25> The coating film according to any one of <19> to <24>, wherein a content mass ratio of the carbon dioxide per $m^2$ of the deposit containing a fiber ($\gamma$) to the fiber per $m^2$ of the deposit containing a fiber ($\alpha$), $[(\gamma)/(\alpha)]$, is 1 or more and 45 or less.
<26> A kit for forming a coating film comprising the following composition (B):

Composition (B): a composition containing carbon dioxide gas; and
a coating film composed of a deposit comprising a fiber or a composition for forming a coating film.

<27> The kit for forming a coating film according to <26>, further comprising the following composition (A):
Composition (A): a composition comprising an amphiphilic molecule and having a lamellar structure.
<28> Use of the kit according to <26> or <27> for reducing wrinkles on skin.
<29> A method for reducing wrinkles on skin, the method comprising

(Step I) applying one or two compositions selected from the compositions (A) and (B) to the skin; and
(Step II) applying a coating film composed of a deposit containing a fiber to the skin
in this order or in the reverse order;
Composition (A): a composition comprising an amphiphilic molecule and having a lamellar structure.
Composition (B): a composition comprising carbon dioxide gas.

<30> The method for reducing wrinkles on skin according to <29>, wherein the step II is a step of holding the coating film composed of a deposit containing a fiber for from 4 to 20 hours per day and then peeling off the coating film.
<31> The method for reducing wrinkles on skin according to <29> or <30>, which is repeated for from 3 to 24 weeks.
<32> The method for reducing wrinkles on skin according to any one of <29> to <31>, wherein one or more amphiphilic molecules are selected from (a1-1) ceramides, (a1-2) polyhydric alcohol fatty acid esters having an intramolecular hydroxy group, (a1-3) a surfactant, and (a1-4) a higher alcohol.
<33> The method for reducing wrinkles on skin according to <32>, wherein the component (a1-2) is one or more selected from the group consisting of glycerol monofatty acid esters, glycerol difatty acid esters, sorbitan monofatty acid esters, sorbitan difatty acid esters, and diglycerol difatty acid esters.
<34> The method for reducing wrinkles on skin according to <32> or <33>, wherein the component (a1-3) is an ionic surfactant.
<35> The method for reducing wrinkles on skin according to any one of <32> to <34>, wherein the component (a1-4) is a monovalent C10-24 alcohol.
<36> The method for reducing wrinkles on skin according to any one of <29> to <35>, wherein the composition (A) further comprises one or more selected from (a2) an oily component other than the component (a1), (a3) a polyhydric alcohol and (a4) water.
<37> The method for reducing wrinkles on skin according to any one of <29> to <36>, wherein the composition (B) further comprises one or more selected from the group consisting of (b2) an oil agent, (b3) a polyol, (b4) a surfactant, (b5) a water-soluble thickener and (b6) water.
<38> The method for reducing wrinkles on skin according to <37>, wherein the component (b4) is a nonionic

surfactant having an HLB value of from 3 to 20.

<39> The method of reducing wrinkles on skin according to <37> or <38>, wherein component (b5) is a (meth)acrylic acid-based polymer.

<40> The method for reducing wrinkles on skin according to any one of <29> to <39>, wherein the composition (B) is an aerosol composition in which a stock solution and a propellant are filled in a pressure-resistant container and contains carbon dioxide gas as a propellant.

<41> The method for reducing wrinkles on skin according to any one of <29> to <40>, wherein the coating film composed of a deposit containing a fiber is a coating film formed by electrostatically spraying a composition X containing the following component (c1) and component (c2):

(c1) one or more volatile substances selected from the group consisting of water, alcohols and ketones;
(c2) a film-forming polymer.

<42> The method of reducing wrinkles on skin according to any one of <29> to <41>, wherein the coating film composed of a deposit containing a fiber is a coating film composed of a deposit containing a fiber of a water-insoluble polymer.

<43> The method of reducing wrinkles on skin according to any one of <29> to <42>, wherein the thickness of the fiber is from 10 nm to 3 000 nm with a circle equivalent diameter.

<44> The method of reducing wrinkles on skin according to any one of <29> to <43>, wherein the step II is a step of adhering a coating sheet composed of a deposit containing a fiber to the skin, or a step of forming a coating film composed of a deposit containing a fiber directly onto the skin by electrostatic spraying.

<45> The method for reducing wrinkles on skin according to any one of <29> to <44>, wherein the area of one or two wrinkles selected from large wrinkles (coarse lines) and small wrinkles (fine lines) is reduced by 10% to 30%.

<46> Use of a combination of one or two compositions selected from the group consisting of the following compositions (A) and (B), and a coating film composed of a deposit containing a fiber or a composition for forming a coating film, for reducing wrinkles on skin:

Composition (A): a composition comprising an amphiphilic molecule and having a lamellar structure.
Composition (B): a composition comprising carbon dioxide gas.

## EXAMPLES

Hereinafter, the present invention will be described in detail with reference to Examples, but the present invention is not limited to these Examples.

[Preparation Example 1: Lamellar Lotion (O/W type emulsified cosmetic composition)]

A lamellar lotion was prepared by a conventional method in accordance with the formulation shown in Table 1. It was confirmed that it has a lamellar structure by measuring the dried coating film of the prepared lotion by small-angle X-ray diffraction and then observing repeated diffraction peaks characteristic of the lamellar structure.

Table 1

| Component (mass%) | | Preparation Example 1 |
|---|---|---|
| a1-1 | N-(hexadecyloxyhydroxypropyl)-N-hydroxyethylhexadecanamide | 1.9 |
| a1-2 | Glyceryl monobehenate *1 | 1.1 |
| a1-2 | Polyglyceryl diisostearate -2 | 0.33 |
| a1-3 | N-stearoyl-L-glutamate | 0.59 |
| a1-4 | Cetanol | 0.9 |
| a2 | $\alpha$-olefin oligomer *2 | 2.5 |
| a2 | vaseline *3 | 1 |
| a2 | Neopentyl glycol dicaprate | 2 |
| a2 | Olive oil *4 | 1 |
| a2 | Methylpolysiloxane *5 | 1 |

(continued)

| Component (mass%) | | Preparation Example 1 |
|---|---|---|
| a2 | Methylpolysiloxane *6 | 5 |
| a2 | Methylpolysiloxane/Cross-Linked Methylpolysiloxane Mixture *7 | 0.3 |
| | Methyl-p-hydroxybenzoate | 0.4 |
| | Ethyl-p-hydroxybenzoate | 0.05 |
| | L-arginine | 0.345 |
| | Potassium hydroxide | 0.11 |
| a3 | Glycerol | 16 |
| a3 | 1,3-butylene glycol | 5 |
| a3 | Polyethylene glycol 1540 | 3 |
| | Carboxyvinyl polymer | 0.18 |
| | Xanthan gum | 0.05 |
| a4 | Purified Water | 57.245 |
| Total (mass%) | | 100 |

The symbols in Table 1 indicate the following, respectively.

*1: Sunsoft No.8100-CK (manufactured by Taiyo Chemical Co., Ltd.)
*2: SILKFLO 364 (manufactured by Vantage Specialty Chemicals)
*3: Super White Protopet (manufactured by Sonneborn, LLC.)
*4: Cropure OL-LQ-(JP) (Croda Japan Co., Ltd.)
*5: KF-96A-10CS (manufactured by Shin-Etsu Chemical Co., Ltd.)
*6: KF-96A-50CS (manufactured by Shin-Etsu Chemical Co., Ltd.)
*7: Silicone KSG-16 (manufactured by Shin-Etsu Chemical Co., Ltd.)

[Preparation Example 2: Aerosol cosmetic (propellant: carbon dioxide)]

Among the stock solution components shown in Table 2, a mixture of the components (b2) to (b6) were stirred until uniform at 60°C. To the obtained liquid, the remaining stock solution component was added, and the mixture was stirred until uniform, and then cooled to 30°C, thereby obtaining a stock solution of an aerosol cosmetic composition. One hundred g of stock solution was filled in a glass container, and the viscosity of the stock solution was measured by a BM viscometer (manufactured by Toki Sangyo Co., Ltd.) (rotor: No. 3, rotation speed :12 rpm, measurement time: 1 minute) at 25°C. The viscosity of the stock solution at 25°C is shown in Table 2.

Subsequently, the stock solution obtained above and a propellant (carbon dioxide) were filled in a pressure-resistant container for an aerosol (stem: SM-S74 (hole diameter φ 0.3mm) manufactured by Mitani Valve Co., Ltd., housing: HG-S7008 (hole diameter φ 0.4mm) manufactured by Mitani Valve Co., Ltd., spout: gold crown shoulder cover assembly spout (manufactured by Yoshino Kogyo Co., Ltd.)) so that the pressure in the pressure-resistant container became 0.8 MPa (25°C), thereby obtaining an aerosol cosmetic composition (foam type).

Table 2

| Component (mass%) | | | Preparation Example 2 |
|---|---|---|---|
| Stock solution | b2 | Isotridecyl isononanoate | 2 |
| | b2 | Methylpolysiloxane *8 | 2.02 |
| | b2 | Methylpolysiloxane *9 | 2 |
| | b2 | Methylpolysiloxane *10 | 0.5 |
| | b3 | Polyethylene glycol 1540 | 1 |
| | b3 | Dipropylene glycol | 5 |
| | b3 | 1,3-propanediol | 2 |
| | b4 | Polyoxyethylene hydrogenated castor oil(HLB:14) *11 | 0.5 |
| | b4 | Polyoxyethylene-methylpolysiloxane copolymer (HLB:4.5) *12 | 0.5 |
| | b5 | (Acrylic Acid/Alkyl Acrylate (C10-30)) Copolymer *13 | 0.4 |
| | | Copolymer of lauryl methacrylate and sodium methacrylate crosslinked with ethylene glycol dimethacrylate *14 | 1.25 |
| | | Methyl Parahydroxybenzoate | 0.2 |
| | | Phenoxyethanol | 0.35 |
| | | Nicotinamide | 0.0001 |
| | | Potassium dihydrogen phosphate | 0.23 |
| | | 48 mass% potassium hydroxide solution | 0.57 |
| | | Disodium edetate | 0.05 |
| | | Perfume | 0.1 |
| | b6 | Purified Water | 81.3299 |
| | Total stock solution (mass%) | | 100 |
| Stock solution | | | 97.6 |
| b1 Carbon dioxide gas | | | 2.4 |
| Total of stock solution and carbon dioxide (mass%) | | | 100 |
| Viscosity of the stock solution at 25°C (mPa•s) | | | 4350 |
| pH of the stock solution at 25 °C | | | 6.9 |

The symbols in Table 2 indicate the following, respectively.

*8: KF-96A-10CS (manufactured by Shin-Etsu Chemical Co., Ltd.)
*9: KF-96A-50CS (manufactured by Shin-Etsu Chemical Co., Ltd.)
*10: KF-96A-100CS (manufactured by Shin-Etsu Chemical Co., Ltd.)
*11: Emanon CH-60K (manufactured by Kao Corporation)

*12: KF-6015 (manufactured by Shin-Etsu Chemical Co., Ltd.)
*13: PEMULEN TR-1 (manufactured by Lubrizol)
*14: SofCare ST-G (manufactured by Kao Corporation)

[Preparation Example 3: Original Coating Patch]

Each of the component shown in Table 3 was weighed into a beaker and stirred using a propeller mixer at ordinary temperature for about 12 hours to produce a composition for forming a fiber coating film. By an electrostatic spraying method in accordance with the description of JP-A-2020-90097 with this composition for forming a fiber coating film, a coating film was formed. The coating film was trimmed into a size suitable for covering from the lower part of the eye to the outer end of the eye, to prepare an eye-coating patch containing a fiber deposit. The thickness (circle equivalent diameter) of the fiber of the eye-coating patch was 700 nm, the basis weight 1.3 $g/m^2$, and the fiber weight 1.08 $g/m^2$.

Table 3

| Component (mass%) | | Preparation Example 3 |
|---|---|---|
| c2 | Polyvinylbutyral *15 | 12.0 |
| c4 | Distearyldimonium chloride *16 | 0.5 |
| c3 | 1,3-butylene glycol | 2.0 |
| c1 | 99.5 mass% ethanol solution | 85.5 |
| Total (mass%) | | 100.0 |

The symbols in Table 3 indicate the following, respectively.

*15: Eslec B BM-1 (manufactured by Sekisui Chemical Industry Co., Ltd.)
*16: Varisoft TA100 (manufactured by Evonik Japan Co., Ltd.)

[Test Example 1]

Nine female subjects aged 39-65 years old conducted a series of procedures including the steps 1) to 2) after facial wash every evening, and a step of peeling the eye-coating patch the following morning for three weeks.

Step 1) Apply about 0.4 mL of the lamellar lotion of Preparation Example 1 to spread over the entire face and neck.

Step 2) After the step 1), apply about 0.2 mL of the lamellar lotion of Preparation Example 1 so as not to be spread, from the lower part of the right eye to the outer end of the right eye (lower half of the circular muscle of the right eye), or from the lower part of the left eye to the outer end of the left eye (lower half of the circular muscle of the left eye), and apply the eye-coating patch of Preparation Example 3 so that the fiber deposit comes into contact with the skin. Gently tap with fingers for 15 to 30 seconds from the center to the end of the eye-coating patch to transfer a coating film to the skin, and peel off a release film (previously laminated to the patch) from the patch.

Then, regarding the part from the lower part of the eye to the outer end which received the step 1) only, and the part from the lower part of the eye to the outer end which received the step 1) and step 2), photoshooting was carried out using VISIA-CR(Canfield Scientific) and Mirror software. This photoshooting was implemented in the modes of Standard 1, Standard 2, parallel polarized lighting, and cross-polarized lighting for the right 45 degrees, the left 45 degrees, and the true front angle, and the area shown by the total lines of wrinkles was obtained from the acquired images. Among the detected wrinkles, the area indicated by the total lines of wrinkles was recorded for the wrinkles characterized by 0.28 mm or more in thickness and the wrinkles characterized by the thickness ranging from 0.28 to 0.34 mm. Using the area indicated by the total lines of wrinkles previously measured before the start of the test (the measurement method is the same as described above), these measurements allowed the evaluation of the change in the wrinkle area after 3 weeks elapsed from the start of the test.

The change in area (in units of the vertical axis: $mm^2$) indicated by the total of all detected wrinkle lines is shown in Fig. 3, the change in area (in units of the vertical axis: $mm^2$) indicated by the total wrinkle lines of 0.28 mm thick or more is shown in Fig. 4, and the change in area (in units of the vertical axis: $mm^2$) indicated by the total lines of wrinkle of 0.28 to 0.34 mm thick is shown in Fig. 5. Example 1 in Figs. 3 to 5 is the result of the site from the lower part of the eye to the outer end of the eye in which the step 1) to the step 2) were implemented, and Comparative Example 1 is the result of the region from the lower part of the eye to the outer end of the eye in which only the step 1) was implemented. The p-values were calculated using ANCOVA's test.

[Test Example 2]

Twelve female subjects aged 40-63 years old conducted a series of procedures including the following steps 2-1) to 2-2) every evening after facial wash, and a step of peeling off the eye-coating patch the following morning for 6 weeks.

Step 2-1) Apply about 1 g of the aerosol cosmetic of Preparation Example 2 and rub uniformly so as to spread over the entire face and neck.

Step 2-2) After the step 2-1), apply about 0.2 g of the lamellar lotion of Preparation Example 1 so as not to be spread, to a site from the lower part of the right eye to the outer end of the right eye (the lower half of the circular muscle of the right eye) or from the lower part of the left eye to the outer end of the left eye (the lower half of the circular muscle of the left eye). Thereon, apply the eye-coating patch of Preparation Example 3 so that the fiber deposit comes into contact with the skin. Gently tap with fingers for 15 to 30 seconds from the center to the end of the eye-coating patch to transfer a coating film to the skin, and peel off a release film (previously laminated to the patch) from the patch.

Prior to the start of the test, specialized evaluators conducted a 10-grade visual evaluation, in which a case where no wrinkle was recognized was scored as "0" and a case where wrinkles were most conspicuous was scored as "9" for each of small wrinkle (fine line) and large wrinkle (coarse line) in a site from the lower part of the eye to the outer end of the eye. Subjects with pre-test scores ranging from 3 to 6 were selected for both the state of the small wrinkles (fine lines) and the state of the large wrinkles (coarse lines), and the mean scores for the small wrinkles of the selected subjects were obtained for both the left and right eyes. One week, 2 weeks, 3 weeks, 5 weeks, and 6 weeks after the start of the test, the same evaluation with the one before the start of the test was implemented on each of the site from the lower part of the eye to the outer end of the eye that received only the step 2-1) and the site from the lower part of the eye to the outer end of the eye that received the step 2-1) to the step 2-2). The average score of the small wrinkle state before the start of the test and the mean score of the small wrinkle state after the start of the test were used to evaluate the change in the score of the small wrinkle state, which was based on the mean score before the start of the test.

Fig. 6 shows the change in the score of the small wrinkle state. Example 2 in Fig. 6 is a result for a site from the lower part of the eye to the outer end of the eye subjected to the step 2-1) to the step 2-2), and Comparative Example 2 is a result for a site from the lower part of the eye to the outer end of the eye subjected only to the step 2-1). The p-values were calculated using Signed Rank's test.

[Preparation Example 4: Composition for Forming a Fiber Coating]

Each of the component shown in Table 3 described above was weighed into a beaker and stirred using a propeller mixer at ordinary temperature for about 12 hours to produce a composition for forming a fiber coating.

[Test Example 3]

Twenty four female subjects aged 35 to 64 years old conducted a series of procedures including the following steps 3-1) to 3-2) every evening after facial wash, and a step of peeling off the eye-coating patch, facial wash, and application of the aerosol cosmetic of Preparation Example 2, a commercially available cosmetic water, and a commercially available emulsion the following morning for 13 weeks.

Step 3-1) After uniformly rubbing about 1 g of the aerosol cosmetic of Preparation Example 2 so as to extend over the entire face and the neck, a commercially available cosmetic and a commercially available emulsion are similarly rubbed on the entire face and the neck.

Step 3-2) After the step 3-1), about 0.4 g of lamellar lotion of Preparation Example 1 was applied to only the right half of the face so as not to be stretched, and from above, the composition for forming a fiber coating of Preparation Example 4 was electrostatically sprayed using an electrostatic spraying device 10 having the configuration shown in Fig. 1 and having the appearance shown in Fig. 2. The conditions for electrostatic spraying are as shown below. The thickness (circle equivalent diameter) of the fiber of the fiber coating is 1 000 nm, the basis weight is 28 g/m$^2$, and the fiber weight 23.2 g/m$^2$.

(Electrostatic spray condition)

- Application site: Right half of face
- Application time: 30 seconds
- Applied voltage: 10kV
- Distance between nozzle and object to be applied: about 80 mm

- Discharge rate: 0.1 g/min
- Applicable environment: 20°C, 50%RH

Prior to the start of the study, a photographic evaluation according to the Guideline for Evaluating Antiwrinkle Function "2-7-5-2. Photographic Evaluation" described in the Journal of Japanese Cosmetic Science Society Vol. 30, No. 4, pp. 316-332

(2006) was conducted by a specialist evaluator on the condition of wrinkles at the end of both eyes of each subject (eight-step evaluation with no wrinkles as "0" and with remarkably deep wrinkles as "7". However, if none of the wrinkle-grade standard photographs described in the Guideline were applicable, an intermediate value or a 1/4 value of the score could be introduced). Subjects with pre-test scores within the range of 3-5 were selected, and the mean scores of the selected subjects were obtained for both the left and right outer ends of the eye. After 13 weeks from the start of the test, the same evaluation with the one before the start of the test was implemented on each of the eye end of the left eye subjected to only the step 3-1) of the selected subject and the eye end of the right eye subjected to the step 3-1) to the step 3-2) of the selected subject.

Fig. 7 shows the result on the outer end of the eye (Example 3) of the right eye which received the steps 3-1) to 3-2). Fig. 8 shows the result on the outer end of the eye (Comparative Example 3) of the left eye which received only the step 3-1). p-Values were calculated using Wilcoxon signed rank sum test.

[Explanation of Symbols]

10 Electrostatic spraying device
11 Low-voltage power supply
12 High-voltage power supply
13 Auxiliary electrical circuit
14 Microgear pump
15 Container
16 Nozzle
17 Pipes
18 Flexible conduit
19 Current limit resistor
20 Housing

## Claims

1. A method for producing a coating film on skin, the method comprising

   (Step IB) applying the following composition (B) onto the skin and
   (Step II) applying a coating film composed of a deposit containing a fiber to the skin;
   in this order or in the reverse order:
   Composition (B): a composition comprising carbon dioxide gas.

2. A method for producing a coating film on skin, the method comprising

   (Step IB) applying the following composition (B) to the skin and
   (Step II) applying a coating film composed of a deposit containing a fiber to the skin to which the composition (B) was applied in the step IB;
   Composition (B): a composition comprising carbon dioxide gas.

3. The method for producing a coating film on skin according to claim 1 or 2, further comprising

   (Step IA) applying the following composition (A) onto the skin;
   Composition (A): a composition comprising an amphiphilic molecule and having a lamellar structure.

4. The method for producing a coating film on skin according to claim 3, wherein the amphiphilic molecule is selected from the group consisting of (a1-1) ceramides, (a1-2) polyol fatty acid esters having an intramolecular hydroxy group,

(a1-3) surfactants and (a1-4) higher alcohols.

**5.** The method for producing a coating film on a skin according to any one of claims 1 to 4, wherein the composition (B) further comprises one or more selected from the group consisting of (b2) oil agents, (b3) polyols, (b4) surfactants, (b5) water-soluble thickeners and (b6) water.

**6.** The method for producing a coating film on skin according to any one of claims 1 to 5, wherein the composition (B) is an aerosol composition in which a stock solution and a propellant are filled in a pressure-resistant container and contains carbon dioxide gas as a propellant.

**7.** The method for producing a coating film on skin according to any one of claims 1 to 6, wherein the coating film composed of a deposit containing a fiber is a coating film formed by electrostatically spraying a composition X comprising the following components (c1) and (c2):

(c1) one or more volatile substances selected from the group consisting of water, alcohols and ketones;
(c2) a film-forming polymer.

**8.** The method for producing a coating film on skin according to any one of claims 1 to 7, wherein the step II is a step of sticking a coating sheet made of a deposit containing a fiber to the skin, or a step of forming a coating film composed of a deposit containing a fiber directly on the skin by electrostatic spraying.

**9.** A coating film for application to skin comprising a deposit containing a fiber, and carbon dioxide gas.

**10.** The coating film according to claim 9, further comprising an amphiphilic molecule.

**11.** The coating film according to claim 10, wherein one or two of components selected from amphiphilic molecules and carbon dioxide gas are contained in one or two selected from the group consisting of a space between fibers and the surface of the fiber.

**12.** A kit for forming a coating film comprising the following composition (B):

Composition (B): a composition comprising carbon dioxide gas; and
a coating film composed of a deposit containing a fiber or a composition for forming a coating film.

**13.** The kit for forming a coating film according to claim 12, further comprising the following composition (A):
Composition (A): a composition comprising an amphiphilic molecule and having a lamellar structure.

**14.** Use of the kit according to claim 12 or 13 for reducing wrinkles on skin.

**15.** A method for reducing wrinkles on skin, the method comprising

(Step I) applying one or two compositions selected from the compositions (A) and (B) to the skin; and
(Step II) applying a coating film composed of a deposit containing a fiber to the skin
in this order or in the reverse order;
Composition (A): a composition comprising an amphiphilic molecule and having a lamellar structure.
Composition (B): a composition comprising carbon dioxide gas.

**16.** The method for reducing wrinkles on skin according to claim 15, wherein the step II is a step of holding the coating film composed of a deposit containing a fiber for from 4 to 20 hours per day and then peeling off the coating film.

**17.** The method for reducing wrinkles on skin according to claim 15 or 16, which is repeated for from 3 to 24 weeks.

**18.** The method for reducing wrinkles on skin according to any one of claims 15 to 17, wherein the area of one or two wrinkles selected from large wrinkles (coarse lines) and small wrinkles (fine lines) is reduced by 10% to 30%.

**19.** Use of a combination of one or two compositions selected from the group consisting of the following compositions (A) and (B), and a coating film composed of a deposit containing a fiber or a composition for forming a coating film, for reducing wrinkles on skin:

Composition (A): a composition comprising an amphiphilic molecule and having a lamellar structure.
Composition (B): a composition comprising carbon dioxide gas.

FIG. 1

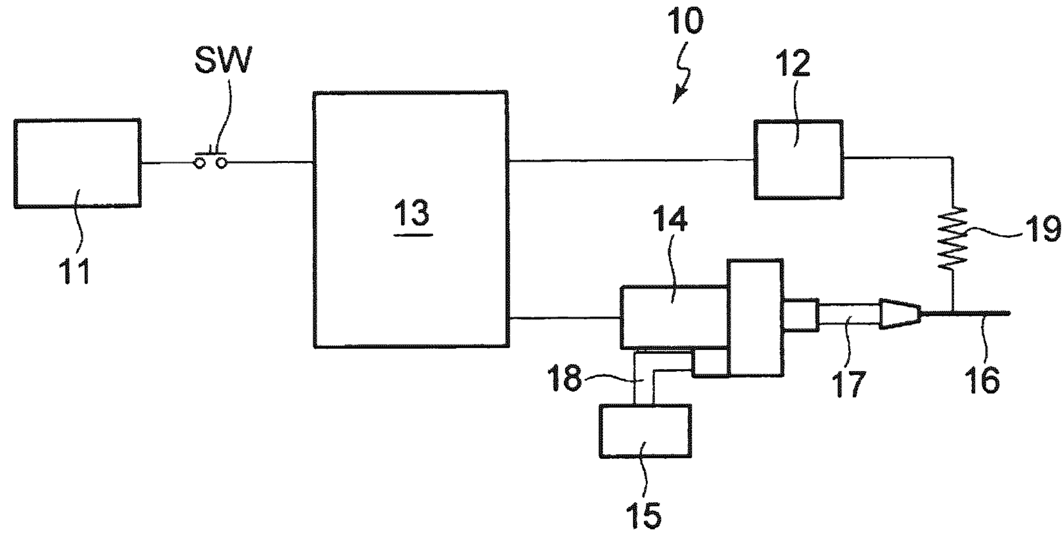


FIG. 2

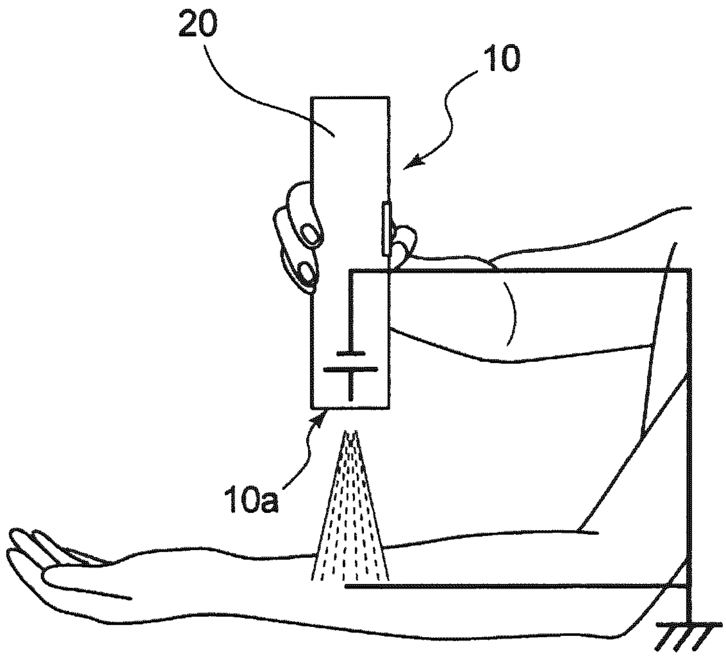

FIG. 3

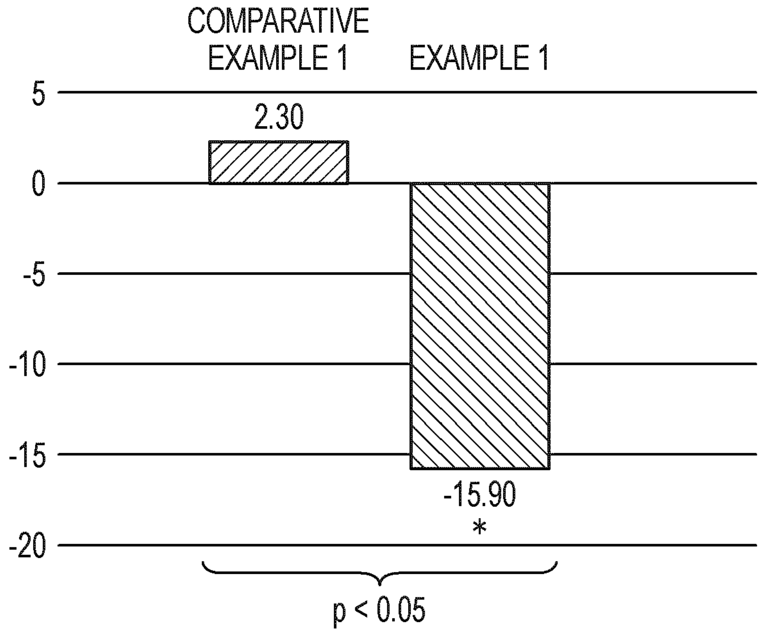
COMPARATIVE EXAMPLE 1
EXAMPLE 1
5
0
-5
-10
-15
-20
2.30
-15.90
*
p < 0.05

FIG. 4

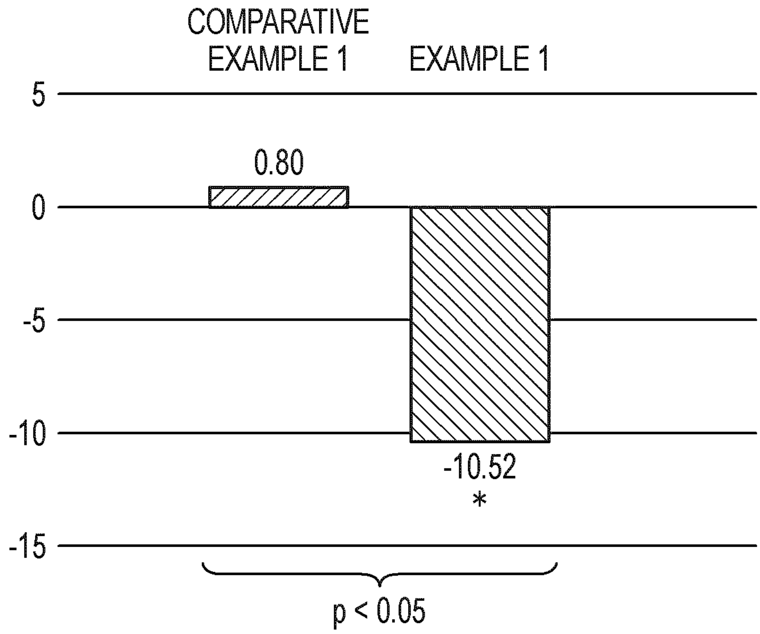
COMPARATIVE EXAMPLE 1
EXAMPLE 1
5
0
-5
-10
-15
0.80
-10.52
*
p < 0.05

FIG. 5

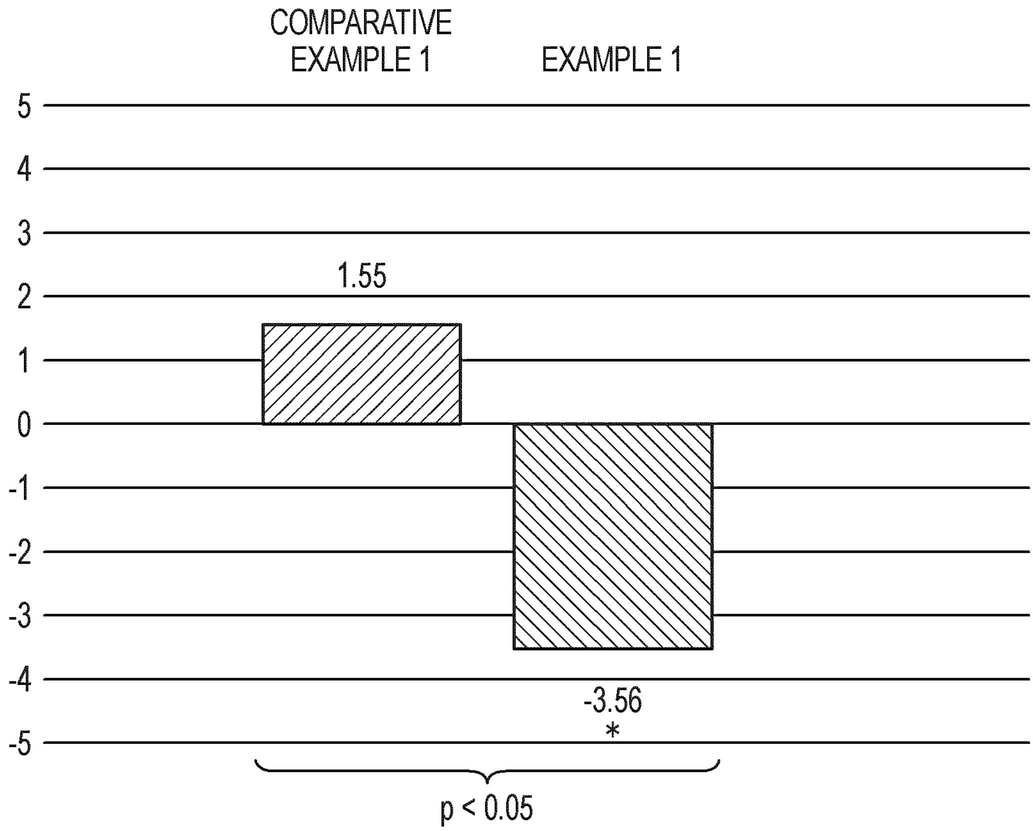

FIG. 6

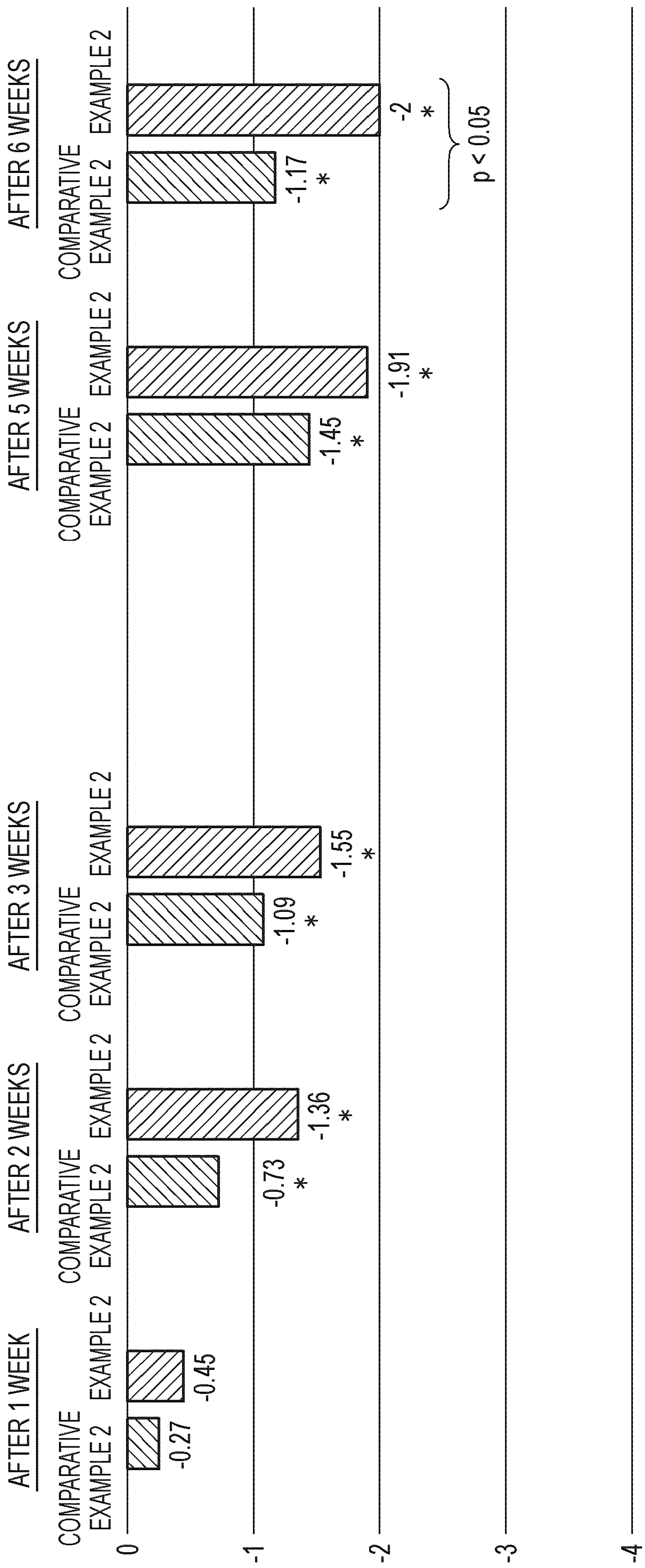
AFTER 1 WEEK
COMPARATIVE EXAMPLE 2
EXAMPLE 2
-0.27
-0.45
AFTER 2 WEEKS
COMPARATIVE EXAMPLE 2
EXAMPLE 2
-0.73 *
-1.36 *
AFTER 3 WEEKS
COMPARATIVE EXAMPLE 2
EXAMPLE 2
-1.09 *
-1.55 *
AFTER 5 WEEKS
COMPARATIVE EXAMPLE 2
EXAMPLE 2
-1.45 *
-1.91 *
AFTER 6 WEEKS
COMPARATIVE EXAMPLE 2
EXAMPLE 2
-1.17 *
-2 *
p < 0.05
0
-1
-2
-3
-4

FIG. 7

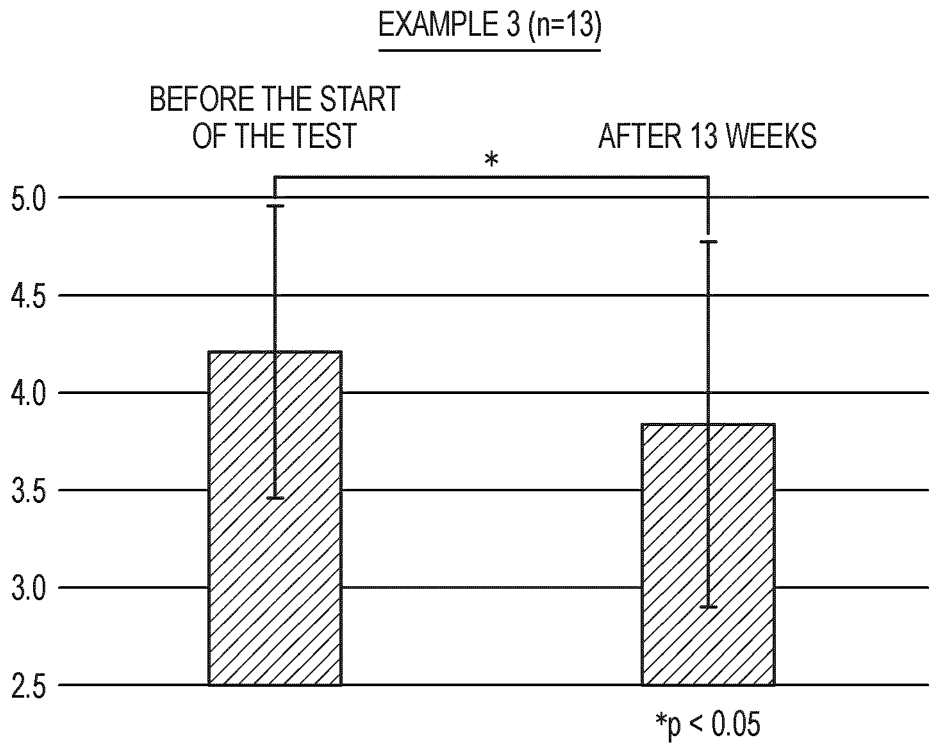
EXAMPLE 3 (n=13)
BEFORE THE START OF THE TEST
AFTER 13 WEEKS
*
5.0
4.5
4.0
3.5
3.0
2.5
*p < 0.05

FIG. 8

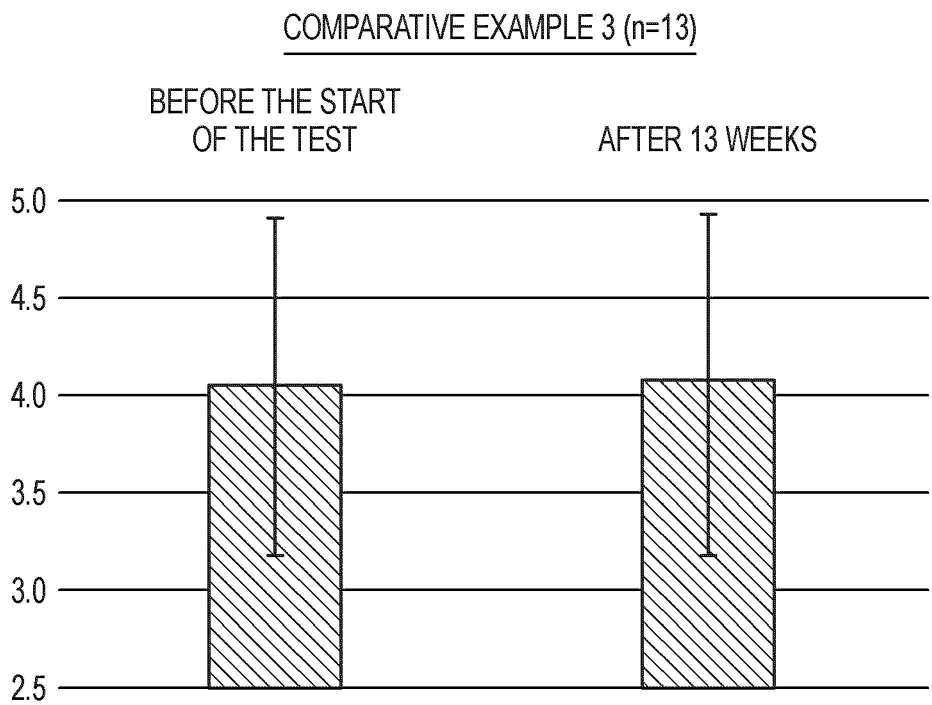
COMPARATIVE EXAMPLE 3 (n=13)
BEFORE THE START OF THE TEST
AFTER 13 WEEKS
5.0
4.5
4.0
3.5
3.0
2.5

**INTERNATIONAL SEARCH REPORT**

International application No. **PCT/JP2021/029753**

**A. CLASSIFICATION OF SUBJECT MATTER**

***A61Q 19/00***(2006.01)i; ***A61Q 19/08***(2006.01)i; ***A61K 8/02***(2006.01)i; ***A61K 8/19***(2006.01)i; ***A61K 8/34***(2006.01)i; ***A61K 8/35***(2006.01)i; ***A61K 8/81***(2006.01)i
FI: A61K8/19; A61K8/34; A61K8/35; A61K8/81; A61K8/02; A61Q19/00; A61Q19/08

According to International Patent Classification (IPC) or to both national classification and IPC

**B. FIELDS SEARCHED**

Minimum documentation searched (classification system followed by classification symbols)

A61Q19/00; A61Q19/08; A61K8/02; A61K8/19; A61K8/34; A61K8/35; A61K8/81

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/156146 A1 (KAO CORP.) 15 August 2019 (2019-08-15) claim 1, test example 1 | 1-19 |
| Y | JP 2016-188199 A (NAGOYA AEROSOL KK) 04 November 2016 (2016-11-04) claims 1, 4 | 1-19 |
| Y | JP 2012-171958 A (KIKOH CORP.) 10 September 2012 (2012-09-10) claims 1, 7, paragraph [0010] | 14-19 |
| A | WO 2017/069079 A1 (KAO CORP.) 27 April 2017 (2017-04-27) entire text | 1-19 |
| A | JP 2016-527218 A (THE PROCTER & GAMBLE CO.) 08 September 2016 (2016-09-08) entire text | 1-19 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 September 2021** | **05 October 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2021/029753**

| Patent document cited in search report | Publication date (day/month/year) | Patent family member(s) | Publication date (day/month/year) |
|---|---|---|---|
| WO 2019/156146 A1 | 15 August 2019 | US 2021/0038530 A1<br>claim 1, test example 1<br>TW 201936159 A | |
| JP 2016-188199 A | 04 November 2016 | (Family: none) | |
| JP 2012-171958 A | 10 September 2012 | (Family: none) | |
| WO 2017/069079 A1 | 27 April 2017 | US 2019/0053602 A1<br>EP 3366270 A1<br>CN 108135789 A<br>KR 10-2018-0051670 A | |
| JP 2016-527218 A | 08 September 2016 | US 2015/0037380 A1<br>WO 2015/009694 A1<br>EP 3021818 A1<br>CN 105377219 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H6345633 A
- JP 2012167061 A
- JP 2018087186 A
- JP 2020090097 A
- JP 2014129306 A
- JP 2017125003 A
- JP 2015105451 A
- JP 2006249025 A


### Non-patent literature cited in the description


- *J. Oleo. Sci.,* 2005, vol. 54 (6), 325-333
- Guideline for Evaluating AntiWrinkle Function. *Journal of Japanese Cosmetic Science Society,* 2006, vol. 30 (4), 316-332
- *Journal of Japanese Cosmetic Science Society,* 2006, vol. 30 (4), 316-332